# EUROPEAN PATENT SPECIFICATION

(11) **EP 4 482 515 B1**
(45) Date of publication and mention of the grant of the patent: **21.10.2026**
(21) Application number: 23759463.5
(22) Date of filing: 23.02.2023
(51) Int. Cl.: A61K 38/29, A61K 9/20, A61K 47/36, A61K 47/38, A61K 9/00, A61P 5/18, A61P 19/00, A61P 19/10

(54) **FORMULATIONS COMPRISING ACID-NEUTRALIZING POLYMER FOR ORAL ADMINISTRATION OF PARATHYROID HORMONE**
FORMULIERUNGEN MIT SÄURENEUTRALISIERENDEM POLYMER ZUR ORALEN VERABREICHUNG VON PARATHORMON
FORMULATIONS COMPRENANT UN POLYMÈRE NEUTRALISANT L'ACIDE POUR L'ADMINISTRATION ORALE D'HORMONE PARATHYROÏDE

(30) Priority: 24.02.2022 US 202263313367 P
(43) Date of publication of application: 01.01.2025
(73) Proprietor: Entera Bio Ltd., 9112002 Jerusalem (IL)
(72) Inventor: BURSHTEIN, Gregory, 7177625 Modiin (IL); ITIN, Constantin, 9076924 Mevaseret-Zion (IL); GALITZER, Hillel, 7681200 Yad Binyamin (IL); SCHWARTZ, Phillip M., 9210806 Jerusalem (IL)
(74) Representative: Nederlandsch Octrooibureau
(86) International application number: PCT/IL2023/050191
(87) International publication number: WO 2023/161933

(56) References cited:
- WO-A1-2006/076692
- WO-A1-2016/128974
- WO-A1-2018/033927
- WO-A2-2006/084164
- CA-A1- 3 183 120
- CN-A- 112 587 496
- CN-B- 108 883 073
- US-A1- 2018 028 622

## Description

### RELATED APPLICATION/S

This application claims the benefit of priority under 35 USC §119(e) of U.S. Provisional Patent Application No. 63/313,367 filed on February 24, 2022.

This application is also related to co-filed PCT International Patent Application having Attorney's Docket No. 95409, entitled "FORMULATIONS COMPRISING ACID-NEUTRALIZING POLYMER FOR ORAL ADMINISTRATION OF ACTIVE AGENTS", which claims the benefit of priority under 35 USC §119(e) of U.S. Provisional Patent Application No. 63/313,363 filed on February 24, 2022.

### FIELD AND BACKGROUND OF THE INVENTION

The present invention, in some embodiments thereof, relates to drug delivery, and more particularly, but not exclusively, to formulations and/or systems for oral administration of therapeutically active agents, such as, but not limited to, a parathyroid hormone.

Human parathyroid hormone (PTH) is secreted by the parathyroid gland as a polypeptide containing 84 amino acids. PTH regulates serum calcium levels by enhancing release of calcium from bones (bone resorption), and by enhancing intestinal absorption and renal reabsorption of calcium. Teriparatide is a recombinant form of the first 34 amino acids of human PTH (hPTH(1-34)), and is used for treatment of osteoporosis. Administration of teriparatide is by subcutaneous injection once per day at a dose of 20 µg [Riek & Towler, Mo Med 2011, 108:118-123].

PTH (including PTH(1-34)) has been reported to enhance bone formation provided that it is administered intermittently, with circulating levels returning to control levels within 3 hours [Martin, J Bone Metab 2014, 21:8-20]. In contrast, prolonged elevated PTH levels deplete bones by enhancing bone resorption.

Oral administration of peptide and/or protein pharmaceuticals is problematic due to degradation of peptides and/or proteins in the digestive system and poor absorption of large molecules. US 2018/028622 describes a tablet comprising a parathyroid hormone, the absorption enhancer SNAC and soybean trypsin inhibitor.

Qi & Ping [J Microencapsulation 2004, 21:37-45] describe administration of enteric microspheres containing insulin with SNAC (sodium 8-N-(2-hydroxybenzoyl)aminocaprylate). The enteric microspheres are for protecting the insulin from digestive enzymes of the stomach and small intestine, and the SNAC is for enhancing absorption.

U.S. Patent Application Publication No. 2011/0142800 describes compositions for oral administration of a protein, comprising a protein having a molecular weight of up to 100,000 Da, a protease inhibitor, and an absorption enhancer, such as SNAC, N-(10-[2-hydroxybenzoyl]amino)decanoic acid (SNAD), 8-[N-(2-hydroxy-4-methoxybenzoyl)amino]caprylic acid (4-MOAC), 8-[N-(2-hydroxy-5-chlorobenzoyl)amino]caprylic acid (5-CNAC) and 4-[(4-chloro-2-hydroxybenzoyl)amino]butanoic acid (4-CNAB) and sodium salts thereof.

International Patent Application Publication No. WO 00/48589 describes solid oral dosage forms comprising a heparin drug in admixture with SNAC or SNAD for facilitating absorption and/or enhancing bioavailability of the heparin drug, wherein the heparin drug is reported to protect the SNAC or SNAD from precipitation during transit through acidic regions of the gastrointestinal (GI) tract.

International Patent Application Publication No. WO 2016/128974 describes a pharmaceutical composition for oral administration comprising a therapeutically active agent, SNAC and at least one antacid compound; as well as a pharmaceutical composition unit dosage form for oral administration which comprises a core comprising a therapeutically active agent and SNAC, and an external layer comprising at least one of an antacid compound and a protease inhibitor.

Buckley et al. [Sci Transl Med 2018, 10:eaar7047] reports that upon oral administration of a tablet comprising the peptide agent semaglutide and SNAC, absorption of the semaglutide takes place in the stomach and is confined to an area in close proximity to the tablet surface, and that the SNAC protects against enzymatic degradation via local buffering actions and only transiently enhances absorption.

Sodium carboxymethylcellulose (CMC) is prepared by modifying cellulose with chloroacetic acid, and may be crosslinked or non-crosslinked. In its crosslinked form, wherein crosslinking is effected by formation of ester bonds between carboxymethyl groups and cellulose, it is commonly referred to as croscarmellose sodium (CCS) or sodium croscarmellose, and is used as a superdisintegrant in pharmaceutical formulations.

Similarly, sodium starch glycolate (SSG) can be prepared by modifying starch with chloroacetic acid to form carboxymethyl groups. The sodium starch glycolate may be further crosslinked by phosphate groups (-O-P(=O)(-O⁻)-O-) between starch backbones, and used as a superdisintegrant.

Additional background art includes Qi et al. [Acta Pharm Sinica 2004, 39:844-848]; International Patent Application Publication Nos. WO 00/50386, WO 01/32130, WO 01/32596, WO 03/045306, WO 03/045331, WO 2006/076692, WO 2007/121471, WO 2010/020978, WO 2012/080471, WO 2016/128970, WO 2016/128971, WO 2016/128972, WO 2016/128973, WO 2016/128974 and WO 2018/033927; Japanese Patent Application Nos. 2005281231 and 2006111558; U.S. Patent No. 8,110,547; and U.S. Patent Application Publication Nos. 2006/0234913, 2007/0087957 and 2013/0224300.

### SUMMARY OF THE INVENTION

According to an aspect of some embodiments of the invention, there is provided a pharmaceutical composition comprising a therapeutically active agent, an absorption enhancer, and a polymer comprising a plurality of alkaline groups. According to embodiments of the present invention, the therapeutically active agent is or comprises a parathyroid hormone.

According to embodiments of the present invention, a concentration of the polymer in the composition is at least 10 weight percent of the total weight of the pharmaceutical composition.

According to embodiments of the present invention, the absorption enhancer is a substituted or non-substituted fatty acid or a salt thereof.

According to an aspect of some embodiments of the invention, there is provided a method of treating a condition treatable by a therapeutically active agent as described herein in a subject in need thereof, the method comprising orally administering to the subject a pharmaceutical composition according to any of the respective embodiments described herein.

According to some of any of the embodiments of the invention, the alkaline groups are carboxylate groups and/or amine groups.

According to some of any of the embodiments of the invention, at least a portion of the alkaline groups are carboxylate groups.

According to some of any of the embodiments of the invention relating to carboxylate groups, at least a portion of the carboxylate groups are in a form of a pharmaceutically acceptable salt.

According to some of any of the embodiments of the invention relating to carboxylate groups, at least a portion of the carboxylate groups are in a form of a sodium salt.

According to some of any of the embodiments of the invention, the absorption enhancer is selected from NAC (8-N-(2-hydroxybenzoyl)aminocaprylic acid), NAD (10-N-(2-hydroxybenzoyl)aminodecanoic acid), 5-CNAC (8-N-(5-chlorosalicyloyl)aminocaprylic acid), 4-MOAC (8-N-(2-hydroxy-4-methoxybenzoyl)aminocaprylic acid), 4-CNAB (4-N-(2-hydroxy-4-chlorobenzoyl)aminobutanoic acid) and salts thereof.

According to some of any of the embodiments of the invention, the absorption enhancer comprises NAC or a salt thereof.

According to some of any of the embodiments of the invention, a concentration of the absorption enhancer is at least 50 weight percent of the total weight of the pharmaceutical composition.

According to some of any of the embodiments of the invention, a concentration of the polymer is at least 20 weight percent of the total weight of the pharmaceutical composition.

According to some of any of the embodiments of the invention, a total concentration of the absorption enhancer and the polymer is at least 80 weight percent of the total weight of the pharmaceutical composition.

According to some of any of the embodiments of the invention, a concentration of the alkaline groups in the pharmaceutical composition is at least 0.1 millimoles per gram.

According to some of any of the embodiments of the invention, the polymer is a crosslinked polymer.

According to some of any of the embodiments of the invention, the polymer comprises a polysaccharide.

According to some of any of the embodiments of the invention relating to a polysaccharide, the polysaccharide is selected from a starch derivative and a cellulose derivative.

According to some of any of the embodiments of the invention, the polymer comprises carboxymethyl groups.

According to some of any of the embodiments of the invention, the polymer is characterized by a pKa in a range of from 1.2 to 7.5.

According to some of any of the embodiments of the invention, the polymer is sodium starch glycolate and/or croscarmellose sodium.

According to some of any of the embodiments of the invention, a Cmax and/or a bioavailability of the pharmaceutical composition upon oral administration is at least 50 % greater than a Cmax and/or a bioavailability of a corresponding composition without the polymer (e.g., a composition comprising the same therapeutically active agent, the same absorption enhancer and optionally other ingredient(s), each in the same amount, but devoid of a polymer comprising a plurality of alkaline groups as described herein in any of the respective embodiments).

According to some of any of the embodiments of the invention, the therapeutically active agent comprises teriparatide.

According to some of any of the embodiments of the invention, the therapeutically active agent comprises eneboparatide (AZP-3601), which is a long acting PTH analog.

According to some of any of the embodiments of the invention, the composition is in a form of a unit dosage form.

According to some of any of the embodiments of the invention relating to a unit dosage form, an amount of the alkaline groups in the unit dosage form is at least 0.03 millimoles.

According to some of any of the embodiments of the invention relating to a unit dosage form, the unit dosage form comprises at least 50 mg of the absorption enhancer.

According to some of any of the embodiments of the invention relating to a unit dosage form, the unit dosage form comprises one or more tablet(s).

According to some of any of the embodiments of the invention, the composition is for use in the treatment of a condition treatable by the therapeutically active agent, the treatment comprising oral administration of the pharmaceutical composition.

According to some of any of the embodiments of the invention relating to treatment of a condition, the condition is selected from osteoporosis, conditions associated with a bone fracture or bone defect, osteoarthritis, and hypoparathyroidism.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE SEVERAL VIEWS OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

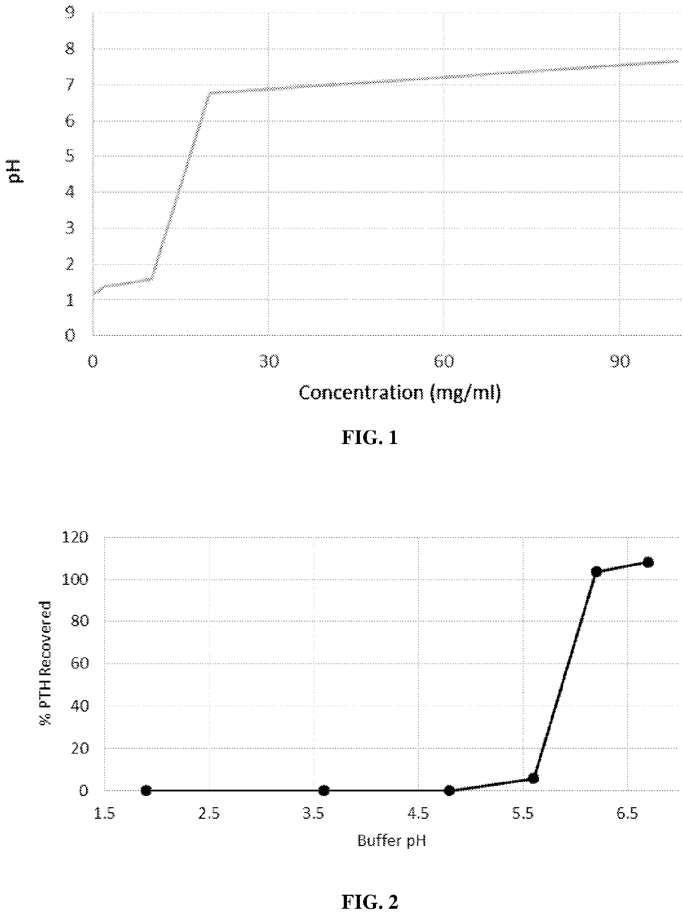

FIG. 1 presents a graph showing the measured pH upon addition of SNAC to an HCl solution (pH 1.2), as a function of SNAC concentration.
FIG. 2 presents a graph showing the percentage of human parathyroid hormone (1-34) (PTH) (initial concentration 0.135 mg/ml) which remained upon incubation with pepsin (150 µg/ml) for 3 minutes at 37 °C, as a function of pH.

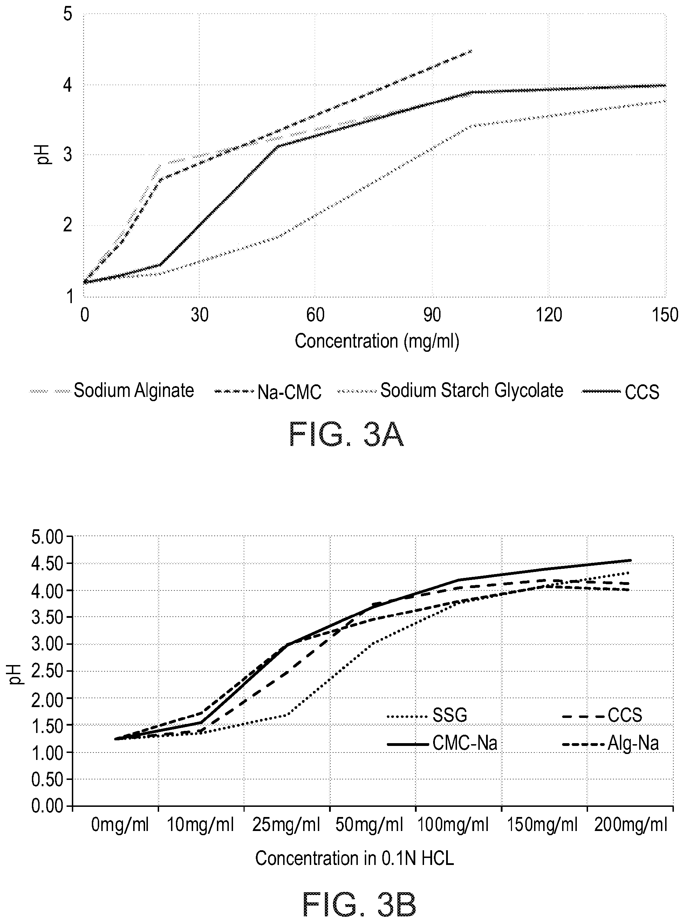

FIGs. 3A-B present graphs showing the pH upon addition of sodium alginate, sodium carboxymethylcellulose (Na-CMC), sodium starch glycolate (SSG) or croscarmellose sodium (CCS) to an HCl solution (pH 1.2), as a function of concentration of the added polymer, measured using MP-103 pH-meter (MRC, Israel) equipped with ELC-10-00 electrode (MRC, Israel) (FIG. 3A) or with a thin electrode HI1083 (HANNA instruments Inc.) (FIG. 3B).

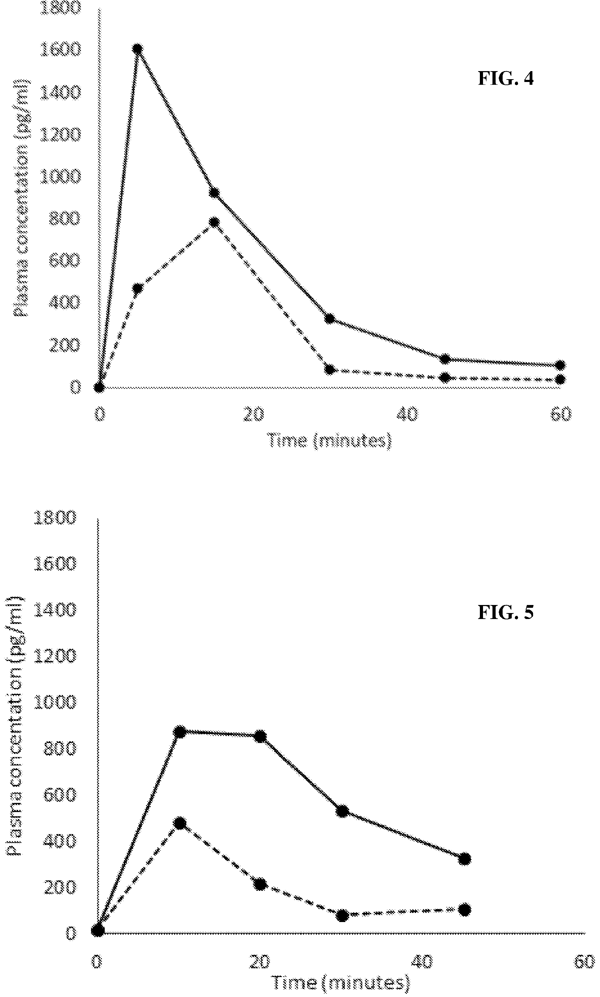

FIG. 4 presents a graph of median plasma concentration of hPTH(1-34) in rats as a function of time following oral administration of mini-tablets comprising 90 µg hPTH(1-34), SNAC and SBTI (soybean trypsin inhibitor), with 20 % sodium starch glycolate (continuous line) or without sodium starch glycolate (dashed line).
FIG. 5 presents a graph of median plasma concentration of hPTH(1-34) in rats as a function of time following oral administration of mini-tablets comprising 90 µg hPTH(1-34) and SNAC, with 30 % sodium starch glycolate (continuous line) or without sodium starch glycolate (dashed line).

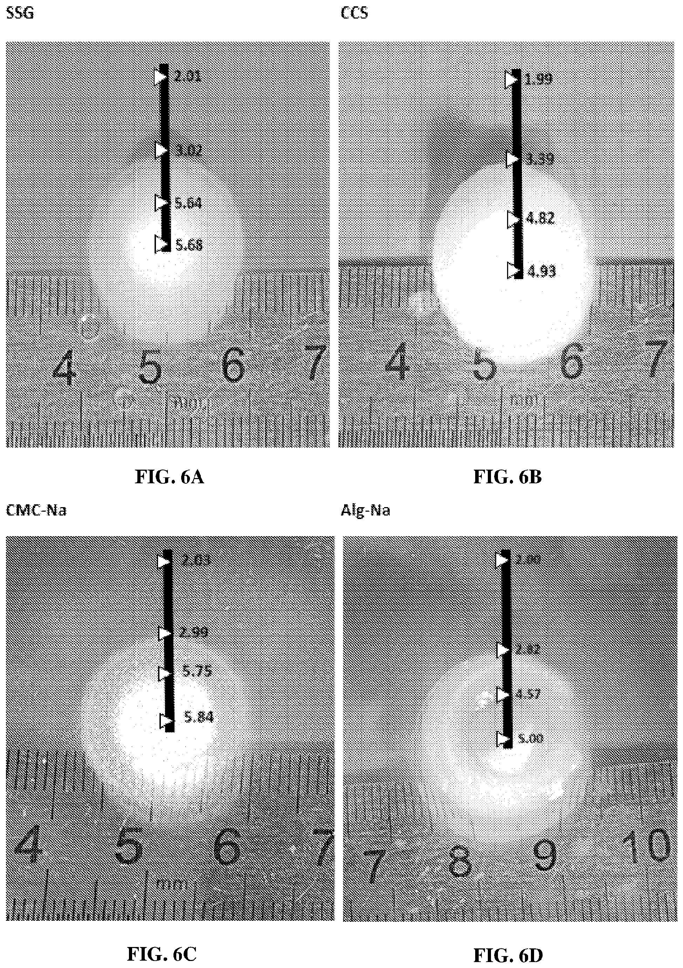

FIGs. 6A-D present photographs of tablets made of the polymers sodium starch glycolate (SSG; FIG. 6A), sodium croscarmellose (CCS; FIG. 6B), sodium carboxymethyl cellulose (CMC-Na; FIG. 6C) and sodium alginate (Alg-Na; FIG. 6D), following swelling in 0.01 M HCl (pH = 2), at room temperature, along with pH values measured at four points for each tablet (white triangles).

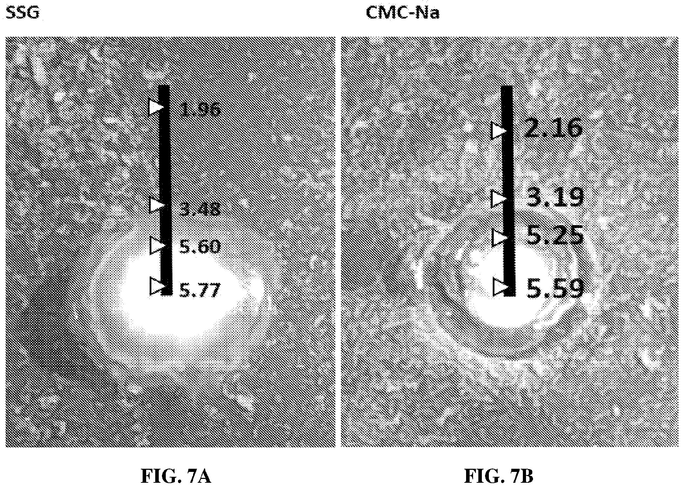

FIGs. 7A-B present photographs of tablets made of the polymers sodium starch glycolate (SSG; FIG. 7A), and sodium carboxymethyl cellulose (CMC-Na; FIG. 7B), following swelling in porcine gastric juice, at room temperature, along with pH values measured at four points for each tablet (white triangles).

### DESCRIPTION OF SPECIFIC EMBODIMENTS OF THE INVENTION

The present invention, in some embodiments thereof, relates to drug delivery, and more particularly, but not exclusively, to formulations and/or systems for oral administration of therapeutically active agents, such as, but not limited to, a parathyroid hormone.

Before explaining at least one embodiment of the invention in detail, it is to be understood that the invention is not necessarily limited in its application to the details set forth in the following description or exemplified by the Examples. The invention is capable of other embodiments or of being practiced or carried out in various ways.

While investigating the enhancement of absorption of therapeutically active agents by an exemplary absorption enhancer such as SNAC (sodium 8-N-(2-hydroxybenzoyl)aminocaprylate), the present inventors have uncovered that the ability of such absorption enhancers to enhance absorption (e.g., as determined by bioavailability and/or Cmax) of therapeutically active agents is adversely affected by the acidic environment of the stomach. The inventors have conceived using basic (alkaline) compounds to enhance the performance of absorption enhancers upon oral administration by neutralizing stomach acid, and uncovered that polymers comprising alkaline groups such as carboxylate are particularly effective at protecting the absorption enhancer from stomach acid, as well as enhancing bioavailability of a therapeutically active agent in compositions comprising the absorption enhancer and the therapeutically active agent.

While reducing the present invention to practice, the inventors have shown that exemplary polymers comprising carboxylate groups produce a local increase in pH in simulated gastric fluid (which increase is capable of reducing protonation of absorption enhancers such as SNAC), and considerably enhance absorption of PTH as compared to corresponding compositions with the same absorption enhancer but without the polymer.

Referring now to the drawings, FIG. 1 shows that the exemplary absorption enhancer SNAC undergoes protonation upon contact with HCl (such as is present in stomach acid).

FIG. 2 shows that increasing pH to about 6 dramatically reduces degradation of peptides by pepsin.

FIGs. 3A-B shows that a variety of carboxylate group-containing polymers increase pH in a concentration-dependent manner.

FIGs. 4 and 5 show that exemplary carboxylate group-containing polymers considerably enhance absorption of a therapeutically active agent in an *in vivo* rat model.

FIGs. 6A-D and 7A-B show the increased local pH inside tablets made of varying carboxylate group-containing polymers, upon hydration in acidic aqueous solutions.

According to an aspect of some embodiments of the invention, there is provided a pharmaceutical composition comprising a therapeutically active agent, an absorption enhancer, and a polymer comprising a plurality of alkaline groups (which for brevity is also referred to herein as an "alkaline group-containing polymer" or "basic polymer" or simply "polymer").

As explained in detail hereinbelow, the alkaline-group containing polymers is also referred to herein as an acid-neutralizing polymer.

According to the present embodiments, the therapeutically active agent is a parathyroid hormone (PTH), as defined herein.

Herein, the terms "parathyroid hormone" and "PTH" (which are used herein interchangeably) refer to a polypeptide (as defined herein) which is a naturally occurring PTH or an analog (e.g., synthetic analog) thereof. The analog may be any derivative of the naturally occurring PTH, such as homologs (as defined herein), fragments (including fragments of a homolog) and substituted derivatives (e.g., comprising a substituent for enhancing stability and/or half-life) of the naturally occurring PTH or homolog and/or fragment thereof, and/or be modified in any other way described herein with respect to polypeptides. Preferably, the analog exhibits a biological activity of the PTH.

In some of any of the embodiments described herein, the PTH is teriparatide. Teriparatide is a fragment (composed of amino acids 1-34, i.e., an N-terminal portion) of the full human parathyroid hormone polypeptide. The term "teriparatide" is used interchangeably herein with the terms "hPTH(1-34)" and "human parathyroid hormone (1-34)".

In some of any of the embodiments described herein, the therapeutically active agent is eneboparatide (AZP-3601), which is a long-acting PTH analog (having increased plasma half-life).

As used herein, the singular form "a", "an" and "the" include plural references unless the context clearly dictates otherwise. For example, the term "a polymer" or "at least one polymer" may include a plurality of polymers, including mixtures thereof.

In some of any of the embodiments described herein, the composition is in a form of a unit dosage form, for example, a tablet or a combination of more than one tablet (e.g., minitablets).

The term "unit dosage form", as used herein, describes physically discrete units, each unit containing a predetermined quantity of one or more active ingredient(s) calculated to produce the desired therapeutic effect, in association with at least one pharmaceutically acceptable carrier, diluent, excipient, or combination thereof. Examples of a suitable unit dosage form include, without limitation, a tablet, a capsule, a lozenge, a dragee, a wafer, a sachet, a patch, an ampoule, a vial, a loaded syringe, and a dose of a metered-dose dispenser. Each unit may optionally comprise discrete subunits (e.g., wherein each subunit is in a form described herein, such as a tablet, capsule, lozenge, dragee, etc.), which may be attached to one another or separate from one another. For example, a unit dosage form according to embodiments of the invention which comprises multiple subunits may optionally be in a form of a unit dosage form comprising discrete subunits bound to one another by a coating and/or matrix, or in a form of a set of dosage forms in a kit (e.g., packaged sets of discrete dosage forms), as described in International Patent Application Publication No. WO 2018/033927.

The pharmaceutical composition and/or unit dosage form according to any of the respective embodiments described herein is preferably formulated to be suitable for oral administration, e.g., as described in more detail herein. Examples of a unit dosage form formulated for oral administration include, without limitation, a tablet, a capsule, a lozenge, a dragee, a wafer, a sachet, an ampoule, and a vial. In exemplary embodiments, the pharmaceutical composition and/or unit dosage form according to any of the respective embodiments described herein is formulated as a tablet or as a plurality of minitablets.

In exemplary embodiments, the pharmaceutical composition and/or unit dosage form according to any of the respective embodiments described herein is devoid of a gastroenteric coating, or otherwise comprises a coating that is dissolvable in gastric fluid and/or immediately (e.g., within less than 5 minutes, less than 2 minutes, or less than 1 minute) releases the composition's content upon contacting gastric fluid. Herein, the phrases "oral administration", "orally administering" and the like refer to any administration via the mouth, preferably by oral ingestion, such as by swallowing (e.g., as opposed to buccal administration).

In some of any of the embodiments described herein, the composition is formulated to allow absorption of the therapeutically active agent in the stomach, for example, by being devoid of an enteric coating, i.e., a coating which prevents release of an agent and dissolves only in the intestines.

Without being bound by any particular theory, it is believed that PTH tends to be poorly absorbed upon oral administration, for example, due to their relatively large molecular weight and/or hydrophilicity (e.g., which inhibits crossing of lipid membranes in the gastrointestinal tract); and therefore, their absorption is particularly susceptible to enhancement by activity of an absorption enhancer (e.g., NAC or a salt thereof) and to increasing absorption enhancer activity by an alkaline group-containing polymer according to any of the respective embodiments described herein. It is further believed that absorption of such polypeptides may be further limited by stomach acid, for example, due to degradation by pH-dependent activity of enzymes such as pepsin (e.g., as exemplified in the Examples section herein), and that a pH increase associated with an alkaline group-containing polymer can enhance absorption of a polypeptide by reducing pepsin activity (in addition to increasing absorption enhancer activity, as described herein).

### Alkaline group-containing polymer:

Herein, the term "polymer" refers a compound having at least 4 repeating (backbone) units (and more preferably at least 10 repeating units, for example, from 4 to 1,000, or from 10 to 1,000 repeating units, with higher numbers of repeating units being also contemplated), the repeating units being identical or similar. The term "polymer" encompasses also a co-polymer, which comprises two or more types of repeating units as described herein, for example, at least 4, or preferably at least 10, repeating units of one type, and at least 4, or preferably at least 10, repeating units of another, different type. The units in a co-polymer can be arranged in any order.

The backbone (repeating) units composing a polymer or copolymer as described herein are also referred to herein interchangeably as monomeric units or simply as monomers.

The alkaline group-containing polymer comprises a plurality (i.e., at least 2) of alkaline groups per molecule. In some of any of the embodiments described herein, the alkaline group-containing polymer comprises at least 4 alkaline groups, or at least 10 alkaline groups, or at least 25 alkaline groups, or at least 50 alkaline groups, or at least 100 alkaline groups, on average, per molecule. In some of any of the embodiments described herein, an average number of the alkaline groups per polymer molecule ranges from about 4 to about 1,000, or from about 10 to about 1,000, or from about 25 to about 1,000, or from about 50 to about 1,000, or from about 100 to about 1,000, with higher upper limit also contemplated for each of these ranges.

In some of any of the embodiments described herein, a mean molecular weight of the alkaline group-containing polymer is at least 1 kDa, or at least 2 kDa, or at least 3 kDa, or at least 5 kDa, or at least 10 kDa. In some of any of the embodiments described herein, a mean molecular weight of the alkaline group-containing polymer ranges from about 1 kDa, or from about 2 kDa, or from about 3 kDa, or from about 5 kDa, or from about 10 kDa, or from about 100 kDa, or from about 200 kDa, or from about 300 kDa, or from about 400 kDa, or from about 500 kDa, or from about 600 kDa, or from about 700 kDa, or from about 800 kDa, or from about 1,000 kDa, or from about 2,000 kDa, and up to about 10,000 kDa or higher, including any intermediate values and subranges therebetween.

Herein, the term "alkaline group" encompasses any functional group capable of accepting a proton in aqueous solution, for example, carboxylate or amine groups. It will be appreciated that an alkaline group may be converted to an acidic group (by protonation), and *vice versa* (by deprotonation), upon contact with a liquid with an appropriate acidity or alkalinity, as these terms are used in the art (e.g., according to pH).

In some of any one of the respective embodiments described herein, the polymer is characterized by a pKa which is at least 1.2, optionally in a range of from 1.2 to 7.5, and optionally in a range of from 1.2 to 5.5 (including any intermediate values and subranges therebetween); for example, from 1.2 to 3 or from 2.5 to 3.5 or from 3 to 4 or from 3.5 to 4.5 or from 4 to 5 or from 4.5 to 5.5, including any intermediate values and subranges therebetween. In some such embodiments, the pKa is at least 2.5, or at least 3, or at least 3.5; for example, from 2.5 to 5.5, or from 3 to 5.5, or from 3.5 to 5.5, or from 4 to 5.5, or from 4.5 to 5.5, including any intermediate values and subranges therebetween.

Herein, the "pKa" of a polymer refers to the pH in an aqueous solution of 0.1 M NaCl, at which 50 % of the acid/base (e.g., carboxylate) groups are protonated at 25 °C.

Carboxylate groups are exemplary alkaline groups according to some embodiments of the invention.

Herein, the term "carboxylate" refers to -C(=O)O⁻ groups (i.e., a carboxylate anion), and does not encompass carboxylic acid groups (-C(=O)OH) unless specifically indicated. It will be appreciated that carboxylate groups may be converted to carboxylic acid groups, and *vice versa,* upon contact with a liquid with an appropriate acidity or alkalinity. For simplicity, some carboxylate group-containing compounds and functional groups described herein (e.g., carboxymethyl, polyacrylic acid) are named as if they were in the carboxylic acid form, but are to be understood as comprising carboxylate groups unless otherwise indicated.

Carboxylate groups according to any of the respective embodiments described herein may optionally be accompanied by a counter-ion to form a salt, for example, a pharmaceutically acceptable salt. A salt (pharmaceutically acceptable salt) of a compound as described herein can alternatively be formed during the synthesis of the compound, e.g., in the course of isolating the compound from a reaction mixture, or re-crystallizing the compound.

Examples of suitable counter-ions which may be comprised by a salt described herein include, without limitation, ammonium, guanidinium, lithium, sodium, potassium, calcium, and magnesium. Sodium is an exemplary counter-ion.

Examples of suitable carboxylate group-containing polymers include, without limitation, salts of alginate, polyacrylic acid, polymethacrylic acid and copolymers of acrylic acid and/or methacrylic acid, and carboxylate group-containing polysaccharide derivatives (e.g., derivatives obtained by oxidation or substitution by a carboxylate-containing group, such as carboxymethyl).

A carboxylate may optionally be comprised by a repeating monomer (backbone unit) which comprises carboxylate, such as a urinate form of a saccharide unit (e.g., mannuronate and guluronate monomers in alginate) and acrylate (e.g., as in a polyacrylic acid salt); comprised by a substituent (e.g., a carboxymethyl substituent, which may optionally be formed by reaction of a polymer with chloroacetic acid) attached to at least a portion of the monomers (e.g., as in carboxymethyl cellulose and sodium starch glycolate); and/or formed by oxidation of at least a portion of the monomers (backbone units), for example, by breaking carbon-carbon bonds and/or by oxidation of a primary carbon (e.g., as in oxidized starch). In some exemplary embodiments, the polymer comprises carboxymethyl groups attached to oxygen atoms.

An amine may optionally be comprised by a repeating monomer (backbone unit) which comprises the amine group; for example, an amino sugar such as a 2-amino-2-deoxysugar (e.g., glucosamine in chitosan), and alkylene imine (e.g., ethylene imine) residues (e.g., as in a polyethyleneimine); and/or comprised by a substituent.

The alkaline group-containing polymer may optionally contain monomers (backbone units) which do not comprise an alkaline group (e.g., acrylate esters, methacrylate esters, acrylamides and methacrylamides), for example, in a copolymer with acrylic acid or methacrylic acid monomers; and/or substituents which do not comprise an alkaline group (e.g., substituted or non-substituted alkyl groups such as methyl, ethyl, hydroxyethyl and hydroxypropyl), for example, in a polymer (e.g., polysaccharide) also substituted by an alkaline group-containing substituent.

The alkaline groups, upon oral administration, are such that are capable of neutralizing an acid by undergoing protonation, to form an acidic group (e.g., a carboxylic acid group or ammonium group), thus increasing the pH of a solution that is in contact with the respective alkaline group-containing polymer.

Without being bound by any particular theory, it is believed that the acid-neutralizing polymer described herein is more suitable than conventional antacids, as the polymer is a large molecule which does not readily diffuse throughout the stomach (thus minimizing dilution of the polymer), which may result in a longer period of efficacy. It is further believed that the polymer described herein provides an additional advantageous effect (which is generally lacking in conventional antacids) of forming a viscous medium which limits influx of stomach acid to the absorption enhancer, thus further reducing inactivation of absorption enhancer by stomach acid; and/or limits diffusion of the absorption enhancer and/or therapeutically active agent throughout the stomach, thus enhancing the effect of the absorption enhancer by maintaining a higher local concentration of absorption enhancer in the vicinity of the therapeutically active agent. In this manner the composition may facilitate absorption of the therapeutically effective agent for a longer time (e.g., by reducing the rate of inactivation of absorption enhancer by protonation and/or by dilution) and/or increase the peak rate of absorption of the therapeutically effective agent (e.g., by providing a higher local concentration of absorption enhancer in active form, as discussed herein). It is further believed that such formation of a viscous medium may facilitate adhesion of the composition to the stomach wall (e.g., by increasing contact area between the soft medium and the stomach wall), thereby promoting absorption via stomach tissue.

In some embodiments of any one of the embodiments described herein, the alkaline group-containing polymer swells upon contact with water (and is also referred to herein in the context of these embodiments as "water-swellable"). In some such embodiments, the polymer is not water-soluble.

Herein, the phrase "swells upon contact with water" refers to an ability of a substance to absorb at least its own weight in water upon contact with water (e.g., pure water) at 37 °C (that is, the substance has a swelling capacity of at least 100 %), and optionally at leasttwice its own weight (a swelling capacity of at least 200 %) or at least 5-fold its own weight (a swelling capacity of at least 500 %) or at least 10-fold its own weight or at least 20-fold its own weight in water.

By "swelling capacity" it is meant that the material is capable of swelling the indicated weight percentage water, of its weight before swelling. The swelling capacity (Qt) can be calculated using the formula: $Qt = \left(Ws-Wd\right) / Ws * 100 % .$ wherein Ws is the weight after swelling and Wd is the dry weight (weight before swelling).

Herein, the term "water-soluble" refers to a compound having a solubility of at least 1 gram per liter in water (e.g., pure water) at 37 °C.

Herein, the term "water-insoluble" refers to a compound having a solubility of less than 1 gram per liter in water (e.g., pure water) at 37 °C.

A typical assay for quantitatively determining solubility of a substance is the "shake-flask method", in which an excess of the tested substance added to a volume (e.g., 100 ml) of solvent (e.g., water) in a container (e.g., flask or vial) and shaken under predetermined conditions (e.g., a temperature of 37 °C) so as to achieve thermodynamic equilibrium Residual solid is then removed (e.g., by filtration and/or centrifugation), and the concentration of dissolved substance is determined by a technique such as HPLC. The concentration may optionally be determined at different time points in order to ascertain that equilibrium has been reached.

Without being bound by any particular theory, it is believed that swelling upon contact with water (e.g., in gastric medium) is advantageous as it enhances penetration of water and maximizes contact between the surrounding solution and alkaline groups upon oral administration. For example, a polymer with a lower degree of penetration of water (e.g., into granules of the polymer) may exhibit slower than optimal neutralization of stomach acid, even if the amount of alkaline groups is high; for example, due to less effective diffusion of acid to the alkaline groups. It is further believed that water solubility, although also effective at maximizing contact between the surrounding solution and alkaline groups, is less desirable than swelling, as dissolution of the polymer disadvantageously results in a less localized effect (e.g., similar to conventional antacids discussed herein).

In some of any of the embodiments described herein, the polymer is a crosslinked polymer.

The polymer can be cross-linked via covalent bonds, by means of cross-linking moieties that are each being covalently attached to, and thereby connects, two or more backbone units in the polymer, and/or via electrostatic bonds, by means of cross-linking moieties (e.g., ions such as cations) that are each being electrostatically attached to, and thereby connects, two or more backbone units in the polymer. The number of cross-linking moieties determines the degree of cross-linking of a cross-linked polymer.

Without being bound by any particular theory, it is believed that crosslinking is advantageous by minimizing dissolution of the polymer (e.g., in saliva and/or stomach acid) which could result in "wastage" of the polymer; and/or by enhancing water-absorption (e.g., by disrupting binding between different polymer chains, thereby facilitating entry of water molecules between the chains). The degree of crosslinking may affect swelling capacity; for example, insufficient crosslinking may be associated with dissolution (as opposed to swelling), and/or to reduced swelling and/or reduced penetration of water. However, excessive crosslinking may reduce swelling capacity by limiting the ability of polymer to expand and provide space for water to enter. The degree of crosslinking should not affect the acid-neutralizing capacity of the polymer. The degree of cross-linking can be selected or pre-determined for each selected polymer, in accordance with its properties, so as to provide the desired hydration level.

In some of any of the embodiments described herein, the polymer comprises a polysaccharide, for example, a crosslinked polysaccharide. The polymer is optionally composed primarily (i.e., more than 50 percent by weight) of glucose units, which may optionally be linked, by glycosidic bonds such as α(1→4) and/or α(1→6) glycosidic bonds (e.g., as in starch) and/or β(1→4) glycosidic bonds (e.g., as in cellulose).

The term "monosaccharide", as used herein and is well known in the art, refers to a simple form of a sugar that consists of a single saccharide molecule which cannot be further decomposed by hydrolysis. Most common examples of monosaccharides include glucose (dextrose), fructose, galactose, and ribose. Monosaccharides can be classified according to the number of carbon atoms of the carbohydrate, i.e., triose, having 3 carbon atoms such as glyceraldehyde and dihydroxyacetone; tetrose, having 4 carbon atoms such as erythrose, threose and erythrulose; pentose, having 5 carbon atoms such as arabinose, lyxose, ribose, xylose, ribulose and xylulose; hexose, having 6 carbon atoms such as allose, altrose, galactose, glucose, gulose, idose, mannose, talose, fructose, psicose, sorbose and tagatose; heptose, having 7 carbon atoms such as mannoheptulose, sedoheptulose; octose, having 8 carbon atoms such as 2-keto-3-deoxy-manno-octonate; nonose, having 9 carbon atoms such as sialose; and decose, having 10 carbon atoms.

Monosaccharides are the building blocks of polysaccharides (such as cellulose and starch).

The term "polysaccharide" as used herein refers to a compound that comprises 10 or more monosaccharide units, as these are defined herein, linked to one another via a glycosyl bond (-O-). The glycosyl bonds between saccharide units in a polysaccharide can all be the same, or can include two or more types of glycosyl bonds, for example, be such that glucose units are linked via α(1→4) and/or α(1→6) glycosidic bonds.

Herein, the term "starch" encompasses polysaccharides composed of amylose (glucose units linked via α(1→4) glycosidic bonds), and amylopectin (glucose units linked via α(1→4) and α(1→6) glycosidic bonds). Different types of starch differ from one another by the ratio between amylose and amylopectin, which typically depends on the source of the starch.

In some of any of the embodiments relating to a polysaccharide, the polysaccharide is a starch derivative and/or a cellulose derivative, that is, starch and/or cellulose (e.g., crosslinked starch and/or crosslinked cellulose) derivatized so as to comprise alkaline (e.g., carboxylate) groups, for example, by being substituted by carboxymethyl groups (e.g., wherein oxygen atoms thereof are attached to carboxymethyl groups). Sodium starch glycolate is an exemplary starch derivative. Croscarmellose sodium is an exemplary cellulose derivative.

Herein, "sodium starch glycolate" refers to any sodium salt of starch (as defined herein) substituted by carboxymethyl groups, and may be crosslinked or non-crosslinked. In some of any of the respective embodiments, the sodium starch glycolate is crosslinked covalently by phosphate groups (-O-P(=O)(-O⁻)-O-), each linking two (or more) saccharide (glucose) units.

Herein, "croscarmellose sodium" refers to any sodium salt of cellulose (as defined herein) substituted by carboxymethyl groups, and crosslinked covalently by ester bonds between carboxymethyl groups and a saccharide unit.

The carboxymethyl groups (or any other alkaline group(s)) can be attached to one or more of the hydroxy substituents of one or more saccharide (e.g., glucose) units composing the polysaccharide, so as to form a -O=CH₂-C(=O)-O⁻ alkaline group which substitute the monosaccharide at one or more positions. In some embodiments, the carboxymethyl groups (or any other alkaline group(s)) are attached at least to the hydroxymethyl group of one or more glucose units that compose the polysaccharide. In some embodiments, the carboxymethyl groups (or any other alkaline group(s)) are attached to at least 10 %, or at least 20 %, or at least 30 %, or at least 40 %, or at least 50 %, or at least 60 %, or at least 70 %, or at least 80 %, or at least 90 % or about all of the saccharide units composing the polysaccharide. In some of these embodiments, one carboxymethyl group (or any other alkaline group) is attached to a saccharide unit comprising same; and in some of these embodiments, the carboxymethyl group (or any other alkaline group) is attached to the hydroxymethyl substituent of the glucose.

In some of any of the embodiments described herein, a concentration of the polymer comprising a plurality of alkaline groups (according to any of the respective embodiments described herein) in the composition is at least 10 weight percent. In some such embodiments, the concentration of the polymer is at least 15 weight percent. In some embodiments, the concentration of the polymer is at least 20 weight percent. In some embodiments, the concentration of the polymer is at least 25 weight percent. In some embodiments, the concentration of the polymer is at least 30 weight percent. In some embodiments, the concentration of the polymer is at least 35 weight percent. In some embodiments, the concentration of the polymer is at least 40 weight percent. In some embodiments, the concentration of the polymer is at least 50 weight percent. In some embodiments, the concentration of the polymer is at least 60 weight percent. In some embodiments, the concentration of the polymer is at least 70 weight percent. In some embodiments, the concentration of the polymer is at least 80 weight percent. In some embodiments, the concentration of the polymer is at least 90 weight percent.

By "weight percent" (or w/w %, or % wt.) it is meant the weight of the indicated substance out of the total weight of a composition comprising same.

In some of any of the embodiments described herein, a concentration of the polymer comprising a plurality of alkaline groups (according to any of the respective embodiments described herein) in the composition ranges from 10 to 90 weight percent, of the total weight of the composition, or from 10 to 80, or from 10 to 70, or from 10 to 50, or from 10 to 40, or from 10 to 30, or from 20 to 90, or from 20 to 80, or from 20 to 70, or from 20 to 60, or from 20 to 50, or from 20 to 40, or from 20 to 30, or from 30 to 90, or from 30 to 80, or from 30 to 70, or from 30 to 60, or from 30 to 50, or from 30 to 40, or from 40 to 90, or from 40 to 80, or from 40 to 70, or from 40 to 60, or from 40 to 50, or from 50 to 90, or from 50 to 80, or from 50 to 70, or from 50 to 60, or from 60 to 90, or from 60 to 80, or from 60 to 70, or from 80 to 90, or from 70 to 80, or from 80 to 90, weight percent, of the total weight of the composition, including any intermediate values and subranges therebetween.

Without being bound by any particular theory, it is believed that a relatively high concentration of water-absorbing (e.g., water-swellable) polymers such as sodium starch glycolate and croscarmellose sodium (e.g., relative to compositions which utilize such polymers as disintegrants) is associated with generation of a viscous medium upon contact with an aqueous solution (e.g., stomach acid) which limits diffusion in the vicinity of the composition, which is significantly different than their effect (e.g., promotion of disintegration of a solid composition) at lower concentrations.

Compositions which utilize such polymers as disintegrants further differ from compositions according to some embodiments of the invention in that disintegrants are normally used in compositions which also comprise a large proportion of insoluble components such as microcrystalline cellulose (e.g., as opposed to compositions comprising large proportions of water soluble components such as SNAC); for example, because the mechanism of the disintegrant involves applying internal forces to a solid insoluble component and/or because a large proportion of water soluble component(s) renders a composition readily soluble, such that a disintegrant would be redundant.

In some of any of the embodiments relating to a polysaccharide described herein, the polysaccharide is characterized by a degree of substitution which is at least 0.1; for example, in a range of from 0.1 to 2 or from 0.1 to 1.5 or from 0.1 to 1 or from 0.1 to 0.5 or from 0.1 to 0.3 or from 0.1 to 0.2, including any intermediate values and subranges therebetween. In some such embodiments, the degree of substitution is at least 0.2; for example, in a range of from 0.2 to 2 or from 0.2 to 1.5 or from 0.2 to 1 or from 0.2 to 0.5, including any intermediate values and subranges therebetween. In some embodiments, the degree of substitution is at least 0.3; for example, in a range of from 0.3 to 2 or from 0.3 to 1.5 or from 0.3 to 1 or from 0.3 to 0.5, including any intermediate values and subranges therebetween. In some embodiments, the degree of substitution is at least 0.4; for example, in a range of from 0.4 to 2 or from 0.4 to 1.5 or from 0.4 to 1, including any intermediate values and subranges therebetween.

Herein, "degree of substitution" refers to the ratio of functional groups comprising an alkaline group or a conjugate acid thereof, such as a carboxylate or carboxylic acid group (e.g., a carboxymethyl group), to monomers (backbone units) of a polymer (e.g., saccharide units of a polysaccharide).

In some of any of the respective embodiments described herein, a concentration of alkaline groups of the polymer in the composition is at least 0.03 millimoles per gram composition; for example, from 0.03 to 5 millimoles per gram or from 0.03 to 2 millimoles per gram or from 0.03 to 1 millimoles per gram or from 0.03 to 0.5 millimoles per gram or from 0.03 to 0.2 millimoles per gram, including any intermediate values and subranges therebetween. In some embodiments, the concentration of alkaline groups of the polymer is at least 0.1 millimoles per gram composition; for example, from 0.1 to 5 millimoles per gram or from 0.1 to 2 millimoles per gram or from 0.1 to 1 millimoles per gram or from 0.1 to 0.5 millimoles per gram, including any intermediate values and subranges therebetween. In some embodiments, the concentration of alkaline groups of the polymer is at least 0.2 millimoles per gram composition; for example, from 0.2 to 5 millimoles per gram or from 0.2 to 2 millimoles per gram or from 0.2 to 1 millimoles per gram or from 0.2 to 0.5 millimoles per gram, including any intermediate values and subranges therebetween. In some embodiments, the concentration of alkaline groups of the polymer is at least 0.5 millimoles per gram composition; for example, from 0.5 to 5 millimoles per gram or from 0.5 to 2 millimoles per gram or from 0.5 to 1 millimoles per gram, including any intermediate values and subranges therebetween. In some embodiments, the concentration of alkaline groups of the polymer is at least 1 millimoles per gram composition; for example, from 1 to 5 millimoles per gram or from 1 to 2 millimoles per gram, including any intermediate values and subranges therebetween.

In some of any of the embodiments described herein, the composition is a unit dosage form composition, and an amount of alkaline groups of the polymer in the unit dosage form (which correlates with the acid-neutralizing capacity of the polymer in the unit dosage form) is at least 0.03 millimoles; for example, from 0.03 to 10 millimoles or from 0.03 to 3 millimoles or from 0.03 to 1 millimoles or from 0.03 to 0.3 millimoles, including any intermediate values and subranges therebetween. In some embodiments, the amount of alkaline groups in the unit dosage form is at least 0.1 millimoles; for example, from 0.1 to 10 millimoles or from 0.1 to 3 millimoles or from 0.1 to 1 millimoles, including any intermediate values and subranges therebetween. In some embodiments, the amount of alkaline groups in the unit dosage form is at least 0.3 millimoles; for example, from 0.3 to 10 millimoles or from 0.3 to 3 millimoles, including any intermediate values and subranges therebetween. In some embodiments, the amount of alkaline groups in the unit dosage form is at least 1 millimoles; for example, from 1 to 10 millimoles, including any intermediate values and subranges therebetween.

As mentioned hereinabove, the alkaline group-containing polymer according to any of the respective embodiments described herein may optionally comprise two or more (e.g., at least 3 or at least 4) distinct polymers (e.g., in admixture) which comprise alkaline groups. Thus, for example, sodium starch glycolate may represent a portion of all the alkaline group-containing polymer, e.g., at least 10 weight percent or at least 20 weight percent or at least 30 weight percent or at least 40 weight percent or at least 50 weight percent or at least 60 weight percent or at least 70 weight percent or at least 80 weight percent or at least 90 weight percent of the alkaline group-containing polymer (according to any of the respective embodiments described herein), the remainder being one or more alkaline group-containing polymers other than sodium starch glycolate. Alternatively or additionally, croscarmellose sodium may represent a portion of all the alkaline group-containing polymer, e.g., at least 10 weight percent or at least 20 weight percent or at least 30 weight percent or at least 40 weight percent or at least 50 weight percent or at least 60 weight percent or at least 70 weight percent or at least 80 weight percent or at least 90 weight percent of the alkaline group-containing polymer (according to any of the respective embodiments described herein), the remainder being one or more alkaline group-containing polymers other than croscarmellose sodium.

It is noted that polymers in general typically comprise a population of molecules of different sizes and with slightly different geometries (e.g., branching patterns and/or sequence of monomers in a copolymer). In accordance with standard practice in the art, such a population of molecules is not considered to represent "distinct polymers". Rather, "distinct polymers" refers to chemical differences, such as polymers composed of different monomers (backbone units) and/or crosslinkers and/or of different degree of cross-linking.

The effect of the polymer on absorption of the therapeutically active agent may optionally be determined by comparing absorption upon oral administration of a composition according to embodiments of the invention with a corresponding composition comprising the same amount of other ingredients other than the alkaline group-containing polymer (e.g., when the alkaline group-containing polymer is no more than 50 weight percent of the composition according to embodiments of the invention), such that the total mass is lower (due to the absence of alkaline group-containing polymer). In some such embodiments, both compositions are in a form of a tablet, and the tablets have the same cross-section (e.g., diameter of a circular cross-section), and the tablet with a lower weight is thinner in the axis perpendicular to the cross-section of the tablet.

Alternatively, the effect of the polymer on absorption of the therapeutically active agent may optionally be determined by comparing absorption upon oral administration of a composition according to embodiments of the invention with a corresponding composition comprising absorption enhancer instead of the alkaline group-containing polymer (e.g., the corresponding composition being identical in all aspects except for the presence of additional absorption enhancer instead of the polymer), such that the total mass of the compositions is the same (e.g., when the alkaline group-containing polymer is more than 50 weight percent of the composition according to embodiments of the invention).

In some embodiments of any one of the embodiments described herein, a Cmax and/or bioavailability of the composition (i.e., of the therapeutically active agent in the composition) upon oral administration is at least 20 % higher than (120 % of the level of) a Cmax and/or bioavailability of a corresponding composition without the alkaline group-containing polymer. In some embodiments, the Cmax and/or bioavailability is at least 50 % higher than (150 % of the level of) the Cmax and/or bioavailability upon oral administration of a corresponding composition without the alkaline group-containing polymer. In some embodiments, the Cmax and/or bioavailability is at least twice (200 % of the level of) the Cmax and/or bioavailability upon oral administration of a corresponding composition without the alkaline group-containing polymer. In some embodiments, the Cmax and/or bioavailability is at least four-fold (400 % of the level of) the Cmax and/or bioavailability upon oral administration of a corresponding composition without the alkaline group-containing polymer. In some embodiments, the Cmax and/or bioavailability is at least six-fold (600 % of the level of) the Cmax and/or bioavailability upon oral administration of a corresponding composition without the alkaline group-containing polymer. In some embodiments, the Cmax and/or bioavailability is at least ten-fold (1000 % of the level of) the Cmax and/or bioavailability upon oral administration of a corresponding composition without the alkaline group-containing polymer.

Cmax and/or bioavailability may optionally be determined by administering the composition orally to subjects (e.g., human subjects) and determining a level of the therapeutically active agent in the blood at frequent intervals by taking blood samples.

Bioavailability may be determined by comparing a ratio of an area under the curve upon oral administration (e.g., over the course of 12 or 24 hours) to an area under curve over the same time period for intravenously administered therapeutically active agent, using standard techniques (e.g., data processing algorithms) known in the art. The therapeutically active agent may be injected at a lower dose (e.g., for safety reasons) and the areas under curve normalized to the total amount administered.

### Absorption enhancer:

According to preferred embodiments of any of the embodiments described herein, the composition according to any of the respective embodiments described herein comprises an effective amount of an absorption enhancer, i.e., an amount of absorption enhancer effective for enhancing absorption of the therapeutically active agent in the composition.

Herein, the term "absorption enhancer" refers to a compound known to enhance absorption of macromolecular drugs (e.g., compounds having a molecular weight of at least 1 kDa) from the gastrointestinal tract into the circulation upon oral administration of the drug. The person skilled in the art will be aware of many such absorption enhancers.

In some of any of the embodiments described herein, the absorption enhancer is a fatty acid, optionally with a terminal N-(2-hydroxybenzoyl)amino group (at the omega position, i.e., the terminus distal from the carboxylate group of the fatty acid), or a salt thereof (e.g., a monosodium or disodium salt). Alternatively, the fatty acid may optionally be a non- substituted fatty acid (e.g., caproic acid, caprylic acid, capric acid, lauric acid, oleic acid and/or stearic acid).

The fatty acid (substituted or non-substituted) is preferably from 4 to 20 carbon atoms in length, optionally from 4 to 18 carbon atoms in length, optionally from 4 to 16 carbon atoms in length, optionally from 4 to 14 carbon atoms in length, optionally from 4 to 12 carbon atoms in length and optionally from 4 to 10 carbon atoms in length, including any intermediate values and subranges therebetween. In some of any of the embodiments described herein, the fatty acid is from 6 to 20 carbon atoms in length, optionally from 6 to 18 carbon atoms in length, optionally from 6 to 16 carbon atoms in length, optionally from 6 to 14 carbon atoms in length, optionally from 6 to 12 carbon atoms in length, optionally from 6 to 10 carbon atoms in length, and optionally from 8 to 10 carbon atoms in length, including any intermediate values and subranges therebetween. The fatty acid moiety may be saturated (e.g., as are caprylic acid in 8-N-(2-hydroxybenzoyl)aminocaprylic acid and decanoic acid in 10-N-(2-hydroxybenzoyl)aminodecanoic acid) or unsaturated (i.e., comprising at least one unsaturated carbon-carbon bond).

Examples of suitable fatty acids (e.g., for substitution by a terminal N-(2-hydroxybenzoyl)amino group) include, without limitation, butanoic acid, caprylic acid and decanoic acid.

The N-(2-hydroxybenzoyl)amino group may optionally be substituted or non-substituted (e.g., on the aromatic ring thereof). Suitable substituents include, for example, halo (optionally chloro) and alkoxy (optionally methoxy). Examples of substituted N-(2-hydroxybenzoyl)amino groups include, without limitation, N-(5-chlorosalicyloyl)amino, N-(4-chloro-2-hydroxybenzoyl)amino, and N-(2-hydroxy-4-methoxybenzoyl)amino.

Examples of suitable absorption enhancers include, without limitation, NAC (8-N-(2-hydroxybenzoyl)aminocaprylic acid) and NAD (10-N-(2-hydroxybenzoyl)aminodecanoic acid) and salts thereof (e.g., monosodium and disodium salts); as well as derivatives thereof (e.g., derivatives substituted by chloro and/or methoxy) such as 5-CNAC (8-N-(5-chlorosalicyloyl)aminocaprylic acid) and 4-MOAC (8-N-(2-hydroxy-4-methoxybenzoyl)aminocaprylic acid) and salts thereof (e.g., monosodium and disodium salts). 4-CNAB (4-N-(2-hydroxy-4-chlorobenzoyl)aminobutanoic acid) and salts thereof (e.g., monosodium and disodium salts) are additional examples of a suitable absorption enhancer.

In some of any of the embodiments described herein, the absorption enhancer is in a form of a salt thereof, for example, a sodium salt. In exemplary embodiments, the sodium salt is a monosodium salt.

In some of any of the embodiments described herein, the absorption enhancer is NAC or NAD, or a salt thereof. In some such embodiments, the absorption enhancer is NAC or a salt thereof.

As shown below, the structure of NAD (depicted as a sodium salt thereof, also referred to as "SNAD") differs from that of NAC (depicted as a sodium salt thereof, also referred to as "SNAC") only in the length of the fatty acid moiety. Additional absorption enhancers related to NAC and NAD, based on different fatty acid lengths, will be readily apparent to the skilled person.

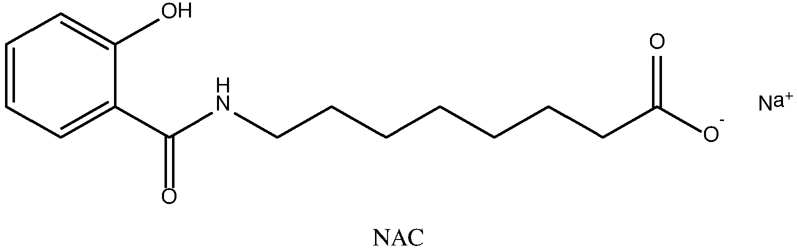

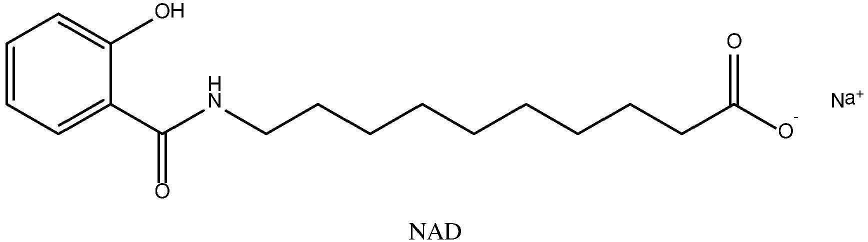

Without being bound by any particular theory, it is believed that many absorption enhancers tend to be more active (at enhancing absorption) in an ionic form (e.g., a carboxylate anion, such as a fatty acid anion) than in a less soluble non-ionic form (e.g., a carboxylic acid, such as a fatty acid), and that overall activity of the absorption enhancer is strongly dependent on concentration of the more active form. For example, the activity of the less soluble non-ionic form may be reduced by precipitation. It is further believed that the amount of absorption enhancer in ionic form upon dissolution following oral administration may be enhanced by providing the absorption enhancer in a form of a salt and/or by controlling a local pH in the vicinity of the absorption enhancer upon oral administration (e.g., by acid neutralization by alkaline groups of a polymer described herein and/or even by other molecules of absorption enhancer).

According to some of any of the embodiments described herein, the absorption enhancer is an ionizable material (compound), and in some of these embodiments, the absorption enhancer is more active in its ionic form.

According to some of any of the embodiments described herein, the absorption enhancer is inactivated by stomach acid, as described herein, and in some of these embodiments, the absorption enhancer is ionizable, is more active in its ionic form, and at least a portion of the absorption enhancer is in a non-ionic (less active) form when contacted with stomach acid (due, e.g., to precipitation).

It is further believed that formation of a viscous medium may enhance the effect of absorption enhancers which are not inactivated by stomach acid, e.g., by maintaining a higher local concentration of absorption enhancer in the vicinity of the therapeutically active agent and/or facilitating adhesion of the composition to the stomach wall (e.g., as described herein).

Additionally or alternatively, the control over local pH associated with the polymer may protect the therapeutically active agent from inactivation by stomach acid (regardless of whether the absorption enhancer is inactivated by stomach acid), for example, inactivation associated with pepsin activity in the presence of a suitably acidic pH (e.g., as discussed elsewhere herein).

According to some of any of the embodiments described herein, the absorption enhancer is not inactivated by stomach acid, for example, it is not ionizable and/or its ionic form and its non-ionic forms exhibit the same activity and/or it is not converted to a less active form when contacted with stomach acid (for example, the absorption enhancer is acidic by itself).

Examples of absorption enhancers which are not expected to be inactivated by stomach acid include, without limitation, alkyl fatty acid esters (e.g., isopropyl myristate); phospholipids (e.g., phosphatidyl choline); quaternary ammonium salts, such as tetraalkyl ammonium salts (e.g., cetyltrimethylammonium salts, such as cetyltrimethylammonium bromide) and alkyl pyridinium salts (e.g., cetylpyridinium salts such as cetylpyridinium chloride); nonionic surfactants such as sorbitan-fatty acid esters (e.g., sorbitan monolaurate, sorbitan monostearate, sorbitan tristearate) and polysorbates (i.e., ethoxylated sorbitan-fatty acid esters, e.g., polysorbate 20, polysorbate 40, polysorbate 60 and polysorbate 80); and N-dodecylcaprolactam.

In some of any one of the embodiments described herein, a concentration of the absorption enhancer (according to any of the respective embodiments described herein) in a composition (according to any of the respective embodiments described herein) is at least 10 weight percent; for example, from 10 to 90 weight percent, or from 10 to 80 weight percent, or from 10 to 70 weight percent, or from 10 to 60 weight percent, or from 10 to 50 weight percent, or from 10 to 40 weight percent, or from 10 to 30 weight percent, including any intermediate values and subranges therebetween. In some such embodiments, the concentration of the absorption enhancer is at least 20 weight percent; for example, from 20 to 90 weight percent, or from 20 to 80 weight percent, or from 20 to 70 weight percent, or from 20 to 60 weight percent, or from 20 to 50 weight percent, or from 20 to 40 weight percent, including any intermediate values and subranges therebetween. In some embodiments, the concentration of the absorption enhancer is at least 30 weight percent; for example, from 30 to 90 weight percent, or from 30 to 80 weight percent, or from 30 to 70 weight percent, or from 30 to 60 weight percent, or from 30 to 50 weight percent, including any intermediate values and subranges therebetween. In some embodiments, the concentration of the absorption enhancer is at least 40 weight percent; for example, from 40 to 90 weight percent, or from 40 to 80 weight percent, or from 40 to 70 weight percent, or from 40 to 60 weight percent, including any intermediate values and subranges therebetween. In some embodiments, the concentration of the absorption enhancer is at least 50 weight percent; for example, from 50 to 90 weight percent, or from 50 to 80 weight percent, or from 50 to 70 weight percent, including any intermediate values and subranges therebetween. In some embodiments, the concentration of the absorption enhancer is at least 60 weight percent; for example, from 60 to 90 weight percent, or from 60 to 80 weight percent, including any intermediate values and subranges therebetween. In some embodiments, the concentration of the absorption enhancer is at least 70 weight percent; for example, from 70 to 90 weight percent or from 70 to 80 weight percent, including any intermediate values and subranges therebetween. In some embodiments, the concentration of the absorption enhancer is at least 80 weight percent; for example, from 80 to 90 weight percent, including any intermediate values and subranges therebetween.

In some of any of the aforementioned embodiments, the absorption enhancer is NAC, NAD, 5-CNAC, 4-MOAC and/or 4-CNAB, or a salt thereof (e.g., a sodium salt thereof).

In some of any one of the embodiments described herein, a total concentration of the absorption enhancer (according to any of the respective embodiments described herein) and the polymer comprising the alkaline groups (according to any of the respective embodiments described herein) is at least 80 weight percent (e.g., from 80 to 100 weight percent, including any intermediate values and subranges therebetween). In some such embodiments, the concentration of the absorption enhancer is at least 10 weight percent or at least 20 weight percent or at least 30 weight percent or at least 40 weight percent or at least 50 weight percent or at least 60 weight percent or at least 70 weight percent (e.g., according to any of the respective embodiments described herein). Alternatively or additionally, in some embodiments, the concentration of the polymer comprising the alkaline groups is at least 10 weight percent or at least 20 weight percent or at least 30 weight percent or at least 40 weight percent or at least 50 weight percent or at least 60 weight percent or at least 70 weight percent (e.g., according to any of the respective embodiments described herein). In some of any of the aforementioned embodiments, the absorption enhancer is NAC, NAD, 5-CNAC, 4-MOAC and/or 4-CNAB, or a salt thereof (e.g., a sodium salt thereof).

In some of any one of the embodiments described herein, a total concentration of the absorption enhancer (according to any of the respective embodiments described herein) and the polymer comprising the alkaline groups (according to any of the respective embodiments described herein) is at least 90 weight percent (e.g., from 90 to 100 weight percent, including any intermediate values and subranges therebetween). In some such embodiments, the concentration of the absorption enhancer is at least 10 weight percent or at least 20 weight percent or at least 30 weight percent or at least 40 weight percent or at least 50 weight percent or at least 60 weight percent or at least 70 weight percent or at least 80 weight percent (e.g., according to any of the respective embodiments described herein). Alternatively or additionally, in some embodiments, the concentration of the polymer comprising the alkaline groups is at least 10 weight percent or at least 20 weight percent or at least 30 weight percent or at least 40 weight percent or at least 50 weight percent or at least 60 weight percent or at least 70 weight percent or at least 80 weight percent (e.g., according to any of the respective embodiments described herein). In some of any of the aforementioned embodiments, the absorption enhancer is NAC, NAD, 5-CNAC, 4-MOAC and/or 4-CNAB, or a salt thereof (e.g., a sodium salt thereof).

In some of any one of the embodiments described herein, a total concentration of the absorption enhancer (according to any of the respective embodiments described herein) and the polymer comprising alkaline groups (according to any of the respective embodiments described herein) is at least 95 weight percent (e.g., from 95 to 100 weight percent, including any intermediate values and subranges therebetween). In some such embodiments, the concentration of the absorption enhancer is at least 10 weight percent or at least 20 weight percent or at least 30 weight percent or at least 40 weight percent or at least 50 weight percent or at least 60 weight percent or at least 70 weight percent or at least 80 weight percent (e.g., according to any of the respective embodiments described herein). Alternatively or additionally, in some embodiments, the concentration of the polymer comprising the alkaline groups is at least 10 weight percent or at least 20 weight percent or at least 30 weight percent or at least 40 weight percent or at least 50 weight percent or at least 60 weight percent or at least 70 weight percent or at least 80 weight percent (e.g., according to any of the respective embodiments described herein). In some of any of the aforementioned embodiments, the absorption enhancer is NAC, NAD, 5-CNAC, 4-MOAC and/or 4-CNAB, or a salt thereof (e.g., a sodium salt thereof).

In some of any one of the embodiments described herein, a total concentration of the absorption enhancer (according to any of the respective embodiments described herein) and the polymer comprising alkaline groups (according to any of the respective embodiments described herein) is at least 98 weight percent. In some such embodiments, the concentration of the absorption enhancer is at least 10 weight percent or at least 20 weight percent or at least 30 weight percent or at least 40 weight percent or at least 50 weight percent or at least 60 weight percent or at least 70 weight percent or at least 80 weight percent (e.g., according to any of the respective embodiments described herein). In some embodiments, the concentration of the polymer comprising the alkaline groups is at least 10 weight percent or at least 20 weight percent or at least 30 weight percent or at least 40 weight percent or at least 50 weight percent or at least 60 weight percent or at least 70 weight percent or at least 80 weight percent (e.g., according to any of the respective embodiments described herein). In some of any of the aforementioned embodiments, the absorption enhancer is NAC, NAD, 5-CNAC, 4-MOAC and/or 4-CNAB, or a salt thereof (e.g., a sodium salt thereof).

In some of any one of the embodiments relating to a unit dosage form described herein, an amount of the absorption enhancer in the unit dosage form is at least 25 mg; for example, from 25 to 1000 mg or from 25 to 500 mg or from 25 to 250 mg or from 25 to 100 mg or from 25 to 50 mg, including any intermediate values and subranges therebetween. In some embodiments, the amount of the absorption enhancer in the unit dosage form is at least 50 mg; for example, from 50 to 1000 mg or from 50 to 500 mg or from 50 to 250 mg or from 50 to 100 mg, including any intermediate values and subranges therebetween. In some embodiments, the amount of the absorption enhancer in the unit dosage form is at least 75 mg; for example, from 75 to 1000 mg or from 75 to 500 mg or from 75 to 250 mg, including any intermediate values and subranges therebetween. In some embodiments, the amount of the absorption enhancer in the unit dosage form is at least 100 mg; for example, from 100 to 1000 mg or from 100 to 500 mg or from 100 to 250 mg, including any intermediate values and subranges therebetween. In some embodiments, the amount of the absorption enhancer in the unit dosage form is at least 150 mg; for example, from 150 to 1000 mg or from 150 to 500 mg or from 150 to 250 mg, including any intermediate values and subranges therebetween. In some embodiments, the amount of the absorption enhancer in the unit dosage form is at least 200 mg; for example, from 200 to 1000 mg or from 200 to 500 mg, including any intermediate values and subranges therebetween. In some embodiments, the amount of the absorption enhancer in the unit dosage form is at least 300 mg; for example, from 300 to 1000 mg or from 300 to 500 mg, including any intermediate values and subranges therebetween. In some of any of the aforementioned embodiments, the absorption enhancer is NAC (8-N-(2-hydroxybenzoyl)aminocaprylic acid) or a salt thereof (e.g., sodium 8-N-(2-hydroxybenzoyl)aminocaprylate).

In some embodiments of any one of the embodiments described herein, a weight ratio of the absorption enhancer to the therapeutically active agent in the composition is at least 1:1 (absorption enhancer: therapeutically active agent), optionally in a range of from 1:1 to 1000:1, or from 1:1 to 500:1, or from 1:1 to 300:1, or from 1:1 to 200:1, or from 1:1 to 100:1, or from 1:1 to 50:1, or from 1:1 to 30:1, or from 1:1 to 20:1, or from 1:1 to 10:1, or from 1:1 to 5:1, or from 1:1 to 3:1, or from 1:1 to 2:1 (absorption enhancer: therapeutically active agent), including any intermediate values and subranges therebetween. In some of the aforementioned embodiments, the absorption enhancer is NAC (8-N-(2-hydroxybenzoyl)aminocaprylic acid) or a salt thereof (e.g., sodium 8-N-(2-hydroxybenzoyl)aminocaprylate).

In some embodiments of any one of the embodiments described herein, a weight ratio of the absorption enhancer to the therapeutically active agent in the composition is at least 2:1 (absorption enhancer: therapeutically active agent), optionally in a range of from 2:1 to 1000:1, or from 2:1 to 500:1, or from 2:1 to 300:1, or from 2:1 to 200:1, or from 2:1 to 100:1, or from 2:1 to 50:1, or from 2:1 to 30:1, or from 2:1 to 20:1, or from 2:1 to 10:1, or from 2:1 to 5:1, or from 2:1 to 3:1 (absorption enhancer: therapeutically active agent), including any intermediate values and subranges therebetween. In some of the aforementioned embodiments, the absorption enhancer is NAC (8-N-(2-hydroxybenzoyl)aminocaprylic acid) or a salt thereof (e.g., sodium 8-N-(2-hydroxybenzoyl)aminocaprylate).

In some embodiments of any one of the embodiments described herein, a weight ratio of the absorption enhancer to the therapeutically active agent in the composition is at least 3:1 (absorption enhancer: therapeutically active agent), optionally in a range of from 3:1 to 1000:1, or from 3:1 to 500:1, or from 3:1 to 300:1, or from 3:1 to 200:1, or from 3:1 to 100:1, or from 3:1 to 50:1, or from 3:1 to 30:1, or from 3:1 to 20:1, or from 3:1 to 10:1, or from 3:1 to 5:1 (absorption enhancer: therapeutically active agent), including any intermediate values and subranges therebetween. In some of the aforementioned embodiments, the absorption enhancer is NAC (8-N-(2-hydroxybenzoyl)aminocaprylic acid) or a salt thereof (e.g., sodium 8-N-(2-hydroxybenzoyl)aminocaprylate).

In some embodiments of any one of the embodiments described herein, a weight ratio of the absorption enhancer to the therapeutically active agent in the composition is at least 5:1 (absorption enhancer: therapeutically active agent), optionally in a range of from 5:1 to 1000:1, or from 5:1 to 500:1, or from 5:1 to 300:1, or from 5:1 to 200:1, or from 5:1 to 100:1, or from 5:1 to 50:1, or from 5:1 to 30:1, or from 5:1 to 20:1, or from 5:1 to 10:1 (absorption enhancer: therapeutically active agent), including any intermediate values and subranges therebetween. In some of the aforementioned embodiments, the absorption enhancer is NAC (8-N-(2-hydroxybenzoyl)aminocaprylic acid) or a salt thereof (e.g., sodium 8-N-(2-hydroxybenzoyl)aminocaprylate).

In some embodiments of any one of the embodiments described herein, a weight ratio of the absorption enhancer to therapeutically active agent in the composition is at least 10:1 (absorption enhancer: therapeutically active agent), optionally in a range of from 10:1 to 1000:1, or from 10:1 to 500:1, or from 10:1 to 300:1, or from 10:1 to 200:1, or from 10:1 to 100:1, or from 10:1 to 50:1, or from 10:1 to 30:1, or from 10:1 to 20:1 (absorption enhancer: therapeutically active agent), including any intermediate values and subranges therebetween. In some of the aforementioned embodiments, the absorption enhancer is NAC (8-N-(2-hydroxybenzoyl)aminocaprylic acid) or a salt thereof (e.g., sodium 8-N-(2-hydroxybenzoyl)aminocaprylate).

In some embodiments of any one of the embodiments described herein, a weight ratio of the absorption enhancer to therapeutically active agent in the composition is at least 20:1 (absorption enhancer: therapeutically active agent), optionally in a range of from 20:1 to 1000:1, or from 20:1 to 500:1, or from 20:1 to 300:1, or from 20:1 to 200:1, or from 20:1 to 100:1, or from 20:1 to 50:1, or from 20:1 to 30:1 (absorption enhancer: therapeutically active agent), including any intermediate values and subranges therebetween. In some of the aforementioned embodiments, the absorption enhancer is NAC (8-N-(2-hydroxybenzoyl)aminocaprylic acid) or a salt thereof (e.g., sodium 8-N-(2-hydroxybenzoyl)aminocaprylate).

In some embodiments of any one of the embodiments described herein, a weight ratio of the absorption enhancer to therapeutically active agent in the composition is at least 30:1 (absorption enhancer: therapeutically active agent), optionally in a range of from 30:1 to 1000:1, or from 30:1 to 500:1, or from 30:1 to 300:1, or from 30:1 to 200:1, or from 30:1 to 100:1, or from 30:1 to 50:1 (absorption enhancer: therapeutically active agent), including any intermediate values and subranges therebetween. In some of the aforementioned embodiments, the absorption enhancer is NAC (8-N-(2-hydroxybenzoyl)aminocaprylic acid) or a salt thereof (e.g., sodium 8-N-(2-hydroxybenzoyl)aminocaprylate).

In some embodiments of any one of the embodiments described herein, a weight ratio of the absorption enhancer to therapeutically active agent in the composition is at least 50:1 (absorption enhancer: therapeutically active agent), optionally in a range of from 50:1 to 1000:1, or from 50:1 to 500:1, or from 50:1 to 300:1, or from 50:1 to 200:1, or from 50:1 to 100:1 (absorption enhancer: therapeutically active agent), including any intermediate values and subranges therebetween. In some of the aforementioned embodiments, the absorption enhancer is NAC (8-N-(2-hydroxybenzoyl)aminocaprylic acid) or a salt thereof (e.g., sodium 8-N-(2-hydroxybenzoyl)aminocaprylate).

In some embodiments of any one of the embodiments described herein, a weight ratio of the absorption enhancer to therapeutically active agent in the composition is at least 100:1 (absorption enhancer: therapeutically active agent), optionally in a range of from 100:1 to 1000:1, or from 100:1 to 500:1, or from 100:1 to 300:1, or from 100:1 to 200:1 (absorption enhancer: therapeutically active agent), including any intermediate values and subranges therebetween. In some of the aforementioned embodiments, the absorption enhancer is NAC (8-N-(2-hydroxybenzoyl)aminocaprylic acid) or a salt thereof (e.g., sodium 8-N-(2-hydroxybenzoyl)aminocaprylate).

In some embodiments of any one of the embodiments described herein, a weight ratio of the absorption enhancer to therapeutically active agent in the composition is at least 200:1 (absorption enhancer: therapeutically active agent), optionally in a range of from 200:1 to 1000:1, or from 200:1 to 500:1, or from 200:1 to 300:1 (absorption enhancer: therapeutically active agent), including any intermediate values and subranges therebetween. In some of the aforementioned embodiments, the absorption enhancer is NAC (8-N-(2-hydroxybenzoyl)aminocaprylic acid) or a salt thereof (e.g., sodium 8-N-(2-hydroxybenzoyl)aminocaprylate).

In some embodiments of any one of the embodiments described herein, a weight ratio of the absorption enhancer to therapeutically active agent in the composition is at least 300:1 (absorption enhancer: therapeutically active agent), optionally in a range of from 300:1 to 1000:1, or from 300:1 to 500:1 (absorption enhancer: therapeutically active agent), including any intermediate values and subranges therebetween. In some of the aforementioned embodiments, the absorption enhancer is NAC (8-N-(2-hydroxybenzoyl)aminocaprylic acid) or a salt thereof (e.g., sodium 8-N-(2-hydroxybenzoyl)aminocaprylate).

In some embodiments of any one of the embodiments described herein, a weight ratio of the absorption enhancer to therapeutically active agent in the composition is at least 500:1, optionally in a range of from 500:1 to 1000:1 (absorption enhancer: therapeutically active agent), including any intermediate values and subranges therebetween. In some of the aforementioned embodiments, the absorption enhancer is NAC (8-N-(2-hydroxybenzoyl)aminocaprylic acid) or a salt thereof (e.g., sodium 8-N-(2-hydroxybenzoyl)aminocaprylate).

### Uses and indications:

In some embodiments of any of the embodiments described herein, the composition according to any of the respective embodiments described herein is for use in the treatment of a condition treatable by the therapeutically active agent (according to any of the respective embodiments described herein), the treatment comprising oral administration of the composition.

According to an aspect of some embodiments of the invention, there is provided a method of treating a condition treatable by oral administration of a therapeutically active agent (according to any of the respective embodiments described herein) in a subject in need thereof, the method comprising orally administering to the subject a composition according to any of the respective embodiments described herein comprising the respective therapeutically active agent (according to any of the respective embodiments described herein).

Condition treatable by the therapeutically active agent include conditions in which activity of PTH is beneficial.

Examples of conditions treatable according to embodiments of the invention include, without limitation, osteoporosis, conditions associated with a bone fracture or bone defect, osteoarthritis, and hypoparathyroidism.

In the context of medical conditions associated with bone fractures, the terms "treating" and "treatment" encompass, for example, substantially healing, at least in part, a bone fracture (e.g., a fracture non-union which does not heal without intervention), substantially increasing a rate at which a bone fracture heals, substantially ameliorating or preventing the appearance of symptoms of a bone fracture (e.g., pain, loss of functionality of a portion of the body, defective bone formation), and preventing or reducing the likelihood of a bone fracture occurring, for example, due to a medical condition (e.g., prophylaxis). Treatment of a bone fracture as described herein may optionally be performed in combination with standard treatments of bone fractures, such as immobilization of bones (e.g., with a cast) and/or surgery.

Examples of conditions associated with a bone fracture include, without limitation, a fracture non-union, any medical condition associated with a stress fracture (optionally the condition is a stress fracture *per se*),

Herein and in the art, the phrase "fracture non-union" refers to a medical condition in which a bone fracture is present, and there is no reasonable expectation that the fracture will heal without intervention.

The skilled person will be readily capable of determining a presence of a fracture non-union.

In some embodiments according to any of the embodiments relating to non-unions, a fracture non-union is determined based on non-consolidation at the fracture site 6 months after the fracture was formed, and/or based on an absence of progress in callus formation at the fracture site at 4 week intervals (e.g., as described by Giannotti et al. [Clin Cases Miner Bone Metab 2013, 10:116-120]).

Herein and in the art, the phrase "stress fracture" refers to a bone fracture caused by repeated stress over time (e.g., by running and/or jumping).

In some embodiments, treating a medical condition associated with a stress fracture comprises increasing a rate at which an existing stress fracture heals.

In some embodiments, treating a medical condition associated with a stress fracture comprises reducing the likelihood of a stress fracture occurring, for example, in a subject susceptible to stress fractures. Examples of subjects susceptible to stress fractures include, without limitation, athletes, runners, soldiers and other people subject to considerable physical exercise.

Herein, the phrase "bone defect" encompasses any missing portion of a bone, including, bone missing due to trauma (e.g., wherein a bone fracture results in a missing bone fragment), surgery (e.g., wherein bone is surgically removed in order to remove cancer cells), resorption of bone, an acquired medical condition (e.g., wherein an acquired medical condition causes a portion of a bone to disappear via resorption) and/or congenital conditions (e.g., wherein a congenitally misshapen bone is associated with one or more defects in the bone structure), a space between a bone and an implant intended to be osseointegrated with the bone (including, but not limited to, an implant anchored in a bone, for example, via a bolt or screw).

Examples of medical conditions involving resorption of bone include, without limitation, bone resorption associated with inflammatory conditions (e.g., periodontitis), which may comprise resorption of bone near the site of inflammation, and resorption of alveolar bone associated with a missing tooth.

Herein, the terms "osseointegration" and "osseointegrated" refer to formation of a direct structural connection (e.g., without intervening connective tissue) between living bone and an implant; and includes, but is not limited to, growth of bone into an implant (e.g., a porous implant), a process also known in the art as "osseoincorporation".

In the context of medical conditions associated with bone defects, the terms "treating" and "treatment" encompass, for example, substantially healing, at least in part, a bone defect (e.g., replacement of at least a portion of missing bone by bone regeneration), substantially increasing a rate at which a bone defect heals (e.g., a rate of bone regeneration), substantially ameliorating or preventing the appearance of symptoms of a bone defect (e.g., pain, loss of functionality of a portion of the body, defective bone formation), and preventing or reducing formation of a bone defect (e.g., prophylaxis), for example, formation of a bone defect by bone resorption. Treatment of a bone defect as described herein may optionally be performed in combination with standard treatments of the respective bone defect.

In some embodiments according to any of the respective embodiments described herein, the medical condition is resorption of alveolar bone. In some of these embodiments, the method or treatment is for preserving and/or regenerating alveolar bone. Examples of resorption of alveolar bone include, without limitation, resorption associated with a missing tooth and resorption associated with inflammation (e.g., periodontitis).

In some embodiments, the method or treatment is for preserving and/or regenerating alveolar bone surrounding a dental implant (e.g., a dental implant which comprises or supports a prosthetic tooth, crown, dental bridge and/or fixed denture), for example, to hold the dental implant in place, thereby increasing the utility of the implant and/or the likelihood of success of the dental implantation. In some embodiments, the method or treatment is effected following implantation of a dental implant, for example, in order to promote regeneration of alveolar bone (e.g., alveolar bone characterized by a bone defect associated with resorption of the bone due to a missing tooth and/or periodontitis). In alternative or additional embodiments, the method or treatment is effected prior to implantation of a dental implant, for example, in order to preserve alveolar bone by preventing or reducing alveolar bone resorption (e.g., upon loss of a tooth, when a significant amount of time is expected to pass before implantation of a dental implant.

In some embodiments according to any of the respective embodiments described herein, the bone defect is in the skull (cranium or lower jawbone). In some embodiments, the bone defect is a calvarial bone defect.

Without being bound by any particular theory, it is believed that bone in the skull (e.g., in the calvaria) is particularly susceptible to poor healing of bone defects, in which promotion of bone growth would be advantageous.

In some embodiments according to any of the respective embodiments described herein, the method and/or treatment comprises promoting osseointegration of an implant, for example, by promoting bone growth in a space between a bone (e.g., calvarial bone) and the implant. The medical condition may optionally be any medical condition for which osseointegration of an implant is beneficial.

Examples of implants for which osseointegration may be promoted include, without limitation, dental implants, bone grafts (e.g., bone allografts), chin implants, craniofacial prostheses (e.g., artificial ears, eyes and/or noses), bone-anchored limb prostheses, bone-anchored hearing aids, and joint prostheses (e.g., for hip and/or knee replacement).

Herein, the term "implant" refers to any device, wherein at least a portion of the device is placed in a subject, and encompasses man-made devices and transplanted tissue, and may comprise synthetic materials, an autograft (e.g., bone harvested from a different region of the subject, such as the iliac crestor chin), an allograft (e.g., bone harvested from an individual other than the subject, optionally a cadaver), a xenograft (e.g., bone from a different species, optionally bovine bone or coral) or any combination thereof. Examples of synthetic material which may be included in an implant (e.g., an implant intended to be osseointegrated) include, without limitation, hydroxyapatite, calcium carbonate, tricalcium phosphate, polymers (e.g., poly(methyl methacrylate), poly(hydroxyethyl methacrylate)), ceramics and metals (e.g., titanium).

In some embodiments of any one of the embodiments described herein relating to treatment of osteoporosis and/or a condition associated with a bone fracture or bone defect, oral administration according to any of the respective embodiments described herein is effected from 1 to 4 times per day. In some such embodiments, the oral administration according to any of the respective embodiments described herein is effected from 1 to 3 times per day. In some embodiments, the oral administration according to any of the respective embodiments described herein is effected once or twice per day. In some embodiments, the oral administration according to any of the respective embodiments described herein is effected once per day.

In some embodiments of any one of the embodiments described herein relating to treatment of a condition associated with a bone fracture or bone defect is effected once per day or less. In some such embodiments, the oral administration is effected once every two days. In some such embodiments, the oral administration is effected twice per week. In some such embodiments, the oral administration is effected once per week or less.

In some embodiments of any one of the embodiments described herein relating to oral administration once per day or less frequently, the treatment is a prophylactic treatment (for preventing or reducing the likelihood and/or size of a bone fracture and/or bone defect), that is, the subject does not necessarily have a bone fracture and/or bone defect at the time of treatment.

In some embodiments, the prophylactic treatment is for stress fractures, for example, in a subject susceptible to stress fractures (e.g., as described herein).

In some embodiments, the prophylactic treatment is for preventing or reducing an alveolar bone defect associated with resorption of alveolar bone, for example, in a subject susceptible to alveolar bone resorption (e.g., as described herein). Subjects afflicted by periodontitis and/or subjects missing a tooth are non-limiting examples of subjects susceptible to alveolar bone resorption.

Without being bound by any particular theory, it is believed that relatively low dosages (e.g., as effected by a relatively low frequency of oral administration) are more suitable than high dosages for prophylactic applications.

Compositions for oral administration (as described herein) may be particularly convenient for indications in which frequent administration of a therapeutically active agent is desirable.

In some embodiments of any one of the embodiments described herein relating to treatment of hypoparathyroidism, the oral administration according to any of the respective embodiments described herein is effected at least twice per day (e.g., from 2 to 6 times per day). In some such embodiments, the oral administration according to any of the respective embodiments described herein is effected at least 3 times per day (e.g., from 3 to 6 times per day). In some embodiments, the oral administration according to any of the respective embodiments described herein is effected at least 4 times per day (e.g., from 4 to 6 times per day).

Without being bound by any particular theory, it is believed that frequent administration of PTH as described in any of the respective embodiments herein provides a relatively steady increase in PTH levels in the body, which is advantageous in the treatment of hypoparathyroidism.

It is expected that during the life of a patent maturing from this application many relevant treatments of conditions by PTH will be developed, and the scope of the phrases "therapeutically active agent" and "condition treatable by... therapeutically active agent" are intended to include all such new technologies *a priori.*

Without being bound by any particular theory, it is believed that compositions according to some embodiments described herein can provide a pharmacokinetic profile characterized by a brief absorption of PTH and acute (rather than chronic) exposure to PTH, which promotes bone growth (e.g., as opposed to chronic exposure, which promotes bone resorption), which is particularly advantageous for some of the indications described herein (e.g., osteoporosis and/or bone fractures).

In some embodiments, the composition (e.g., composition unit dosage form) is formulated such that absorption of the therapeutically active agent following oral administration of the composition is characterized by a Tmax which is 60 minutes or lower. In some embodiments, the Tmax is 50 minutes or lower. In some embodiments, the Tmax is 40 minutes or lower. In some embodiments, the Tmax is 30 minutes or lower. In some embodiments, the Tmax is 20 minutes or lower. In some embodiments, the Tmax is 15 minutes or lower. In some embodiments, the Tmax is 10 minutes or lower.

In some embodiments, the composition (e.g., composition unit dosage form) is formulated such that absorption of the therapeutically active agent following oral administration of the composition is characterized by a ratio of AUC to Cmax which is 3 hours or lower. In some embodiments, the ratio of AUC to Cmax is 2 hours or lower. In some embodiments, the ratio of AUC to Cmax is 90 minutes or lower. In some embodiments, the ratio of AUC to Cmax is 60 minutes or lower. In some embodiments, the ratio of AUC to Cmax is 50 minutes or lower. In some embodiments, the ratio of AUC to Cmax is 40 minutes or lower. In some embodiments, the ratio of AUC to Cmax is 30 minutes or lower. In some embodiments, the ratio of AUC to Cmax is 20 minutes or lower. In some embodiments, the ratio of AUC to Cmax is 15 minutes or lower. In some embodiments, the ratio of AUC to Cmax is 10 minutes or lower.

As used herein the term "AUC" refers to the area under a curve which represents levels of an administered agent in the blood (e.g., plasma levels) as a function of time following administration, and can be determined by measuring plasma levels of the agent at various time points following administration, as exemplified herein.

As used herein the term "Cmax" refers to the maximal concentration of an administered agent in the blood (e.g., plasma levels), and can be determined by measuring levels of the agent at various time points following administration, as exemplified herein.

As used herein the term "Tmax" refers to the time from administration to the time point at which maximal concentration of an administered agent in the blood (e.g., plasma levels) occurs, and can be determined by measuring levels of the agent at various time points following administration, as exemplified herein.

As PTH is normally present to some degree in the blood prior to administration, the area under the baseline levels are excluded from the AUC and Cmax (e.g., by subtracting the baseline level from the measured levels at each time point), such that the AUC and Cmax each represent an aspect of the increase above baseline levels which occurs following administration. The baseline can optionally be determined by measuring levels prior to administration and/or by determining (e.g., by curve-fitting) the baseline to which levels decay after administration. Alternatively or additionally, in embodiments wherein the administered species (e.g., teriparatide) is distinct from endogenous PTH, the measurement of the therapeutically active agent may be selective for the administered agent.

The ratio of AUC to Cmax (i.e., AUC divided by Cmax) will depend on the nature of the pharmacokinetic profile of the composition, particularly on the shape of the curve which represents levels of the therapeutically active agent in the blood (e.g., plasma levels) as a function of time following administration. Pharmacokinetic profiles characterized by a sharp increase and decrease within a brief period of time will tend to have a relatively low ratio of AUC to Cmax, whereas pharmacokinetic profiles characterized by a more gradual increase and decrease over a broader period of time will tend to have a relatively high ratio of AUC to Cmax.

Thus, without being bound by any particular theory, it is believed that a ratio of AUC to Cmax which is 3 hours or lower, as described herein according to any of the respective embodiments, is associated with a relatively sharp increase and decrease of levels of therapeutically active agent in the blood.

The ratio of AUC to Cmax is optionally calculated based on data from multiple administrations of the composition. In such cases, a ratio of AUC to Cmax is preferably calculated for each administration, and then the ratios calculated for each administration may be averaged.

Similarly, the Cmax and/or Tmax are optionally calculated based on data from multiple administrations of the composition. In such cases, a Cmax and/or Tmax value is preferably calculated for each administration, and then the Cmax and/or Tmax values calculated for each administration may be averaged.

Without being bound by any particular theory, it is believed that averaging data (e.g., measured blood levels of therapeutically active agent) from different administrations of therapeutically active agent will frequently result in a broader curve, a lower Cmax value, and larger ratio of AUC to Cmax, than that which is observed after a single administration. Hence, a Cmax value and a ratio of AUC to Cmax calculated for averaged data (as opposed to an average of ratios calculated for each administration, as described hereinabove) is a less accurate indicator of the effect of the composition following administration.

### Formulation of compositions:

Each of the compositions and unit dosage forms described herein optionally consist essentially of the functional ingredients described hereinabove (e.g., a therapeutically active agent, an absorption enhancer, and an alkaline group-containing polymer, according to any of the embodiments described herein in any of the respective sections herein), or alternatively, the composition further comprises suitable pharmaceutically acceptable carriers and/or excipients.

Hereinafter, the phrases "physiologically acceptable carrier" and "pharmaceutically acceptable carrier", which may be interchangeably used, refer to a carrier or a diluent that does not cause significant irritation to an organism and does not abrogate the activity (e.g., biological activity) and properties of the functional ingredient (e.g., a therapeutically active agent). An adjuvant is included under these phrases.

Herein the term "excipient" refers to an inert substance added to a pharmaceutical composition to further facilitate administration of an active ingredient. Examples, without limitation, of excipients include calcium carbonate, calcium phosphate, various sugars and types of starch (e.g., types of non-modified starch), cellulose derivatives, gelatin, vegetable oils and polyethylene glycols.

In some embodiments of any one of the embodiments described herein, the composition is formulated as a solid composition, e.g., a solid unit dosage form. In some embodiments, the composition is formulated as a tablet or as a combination of tablets (e.g., a plurality minitablets). In some embodiments, the composition is formulated as a capsule, as described herein, which comprises, for example, a plurality of minitablets and/or a powder, and which has a coating that is dissolvable in a gastric fluid (e.g., in gastric pH) or otherwise releases the capsule's content immediately (e.g., within less than 5 minutes, or less than 2 minutes or less than 1 minutes) upon contacting a gastric fluid.

Ingredients of the formulation (including, but not limited to, any two or more of the alkaline group-containing polymer, absorption enhancer and therapeutically active agent of any of the respective embodiments described herein) may optionally be mixed in a homogeneous manner or be distributed throughout the composition in a heterogeneous manner.

Techniques for formulation and administration of drugs may be found in "Remington's Pharmaceutical Sciences," Mack Publishing Co., Easton, PA, latest edition.

Pharmaceutical compositions and unit dosage forms of some embodiments of the invention may be manufactured by processes well known in the art, e.g., by means of conventional mixing, dissolving, granulating, dragee-making, levigating, emulsifying, encapsulating, entrapping or lyophilizing processes.

Pharmaceutical compositions and unit dosage forms for use in accordance with some embodiments of the invention may thus be formulated in conventional manner using one or more physiologically acceptable carriers comprising excipients and auxiliaries, which facilitate processing of the active ingredients into preparations which, can be used pharmaceutically.

The pharmaceutical composition and unit dosage forms can be formulated readily by combining the active compounds with pharmaceutically acceptable carriers well known in the art as being suitable for oral administration. Such carriers optionally facilitate formulation of the pharmaceutical composition as tablets (including minitablets), pellets, pills, dragees, capsules, powders, granules, elixirs, tinctures, liquids, gels, syrups, slurries, suspensions, emulsions and the like, for oral administration to a patient. Pharmacological preparations for oral use can be made using a solid excipient, optionally grinding the resulting mixture, and processing the mixture of granules, after adding suitable auxiliaries if desired, to obtain tablets or dragee cores. Pharmaceutical composition and unit dosage forms suitable for oral administration include, but are not limited to, rapid-release, time controlled-release, extended-release, and delayed-release pharmaceutical dosage forms. In some of any of the respective embodiments, a pharmaceutical composition formulated for oral administration is a solid composition, for example, tablet, capsule, powder or granules.

Suitable excipients are, in particular, fillers such as sugars, including lactose, sucrose, mannitol, or sorbitol; cellulose preparations such as, for example, methyl cellulose and/or hydroxypropylmethyl-cellulose; non-modified starches such as maize, wheat, rice and/or potato starch; gelatin; gum tragacanth; and/or physiologically acceptable polymers such as polyvinylpyrrolidone (PVP). If desired, lubricants may be added, such as talc or magnesium stearate.

In some embodiments of any one of the embodiments described herein, any one of the compositions or unit dosage forms described herein (e.g., formulated as a tablet) further comprises a lubricant. In some embodiments, the lubricant is included in a concentration of 5 weight percent or less, optionally 2 weight percent or less, and optionally about 1 weight percent. In some embodiments, the composition or unit dosage form described herein (e.g., formulated as a tablet) consists essentially of the therapeutically active agent (as described herein), absorption enhancer, lubricant, alkaline group-containing polymer, and optional protease inhibitor (as described herein). In some embodiments, the lubricant is magnesium stearate.

The compositions or unit dosage forms described herein (e.g., formulated as a tablet) may alternatively or in addition comprise additives and/or additional agents, such as, for example, antioxidants, solvents, odor absorbers, chelating agents, preservatives, thickeners, colorants or coloring agents (pigments, nacres, water-soluble dyestuffs), dispersants, fillers, flavoring agents and bactericides.

Dragee cores are optionally provided with suitable coatings. For this purpose, concentrated sugar solutions may be used which may optionally contain gum arabic, talc, polyvinylpyrrolidone, carbopol gel, polyethylene glycol, titanium dioxide, lacquer solutions and suitable organic solvents or solvent mixtures. Dyestuffs or pigments may be added to the tablets or dragee coatings for identification or to characterize different combinations of active compound doses.

Pharmaceutical compositions which can be used orally include push-fit capsules made of gelatin as well as soft, sealed capsules made of gelatin and a plasticizer, such as glycerol or sorbitol, pullulan, or HPMC. The push-fit capsules may contain the active ingredients in admixture with filler such as lactose, binders such as starches, lubricants such as talc or magnesium stearate and, optionally, stabilizers. In soft capsules, the active ingredients may be dissolved or suspended in suitable liquids, such as fatty oils, liquid paraffin, or liquid polyethylene glycols. In addition, stabilizers may be added.

In some of any of the embodiments described herein, the pharmaceutical composition and/or unit dosage form according to any of the respective embodiments described herein is devoid of a gastroenteric coating, or otherwise comprises a coating that is dissolvable in the GI tract (e.g., in gastric fluid) and/or which otherwise releases the contents immediately (e.g., within no more than 5 minutes, or no more than 2 minutes or no more than one minute) upon contacting a gastric fluid.

Pharmaceutical compositions suitable for use in context of some embodiments of the invention include compositions wherein the therapeutically active agent is contained in an amount effective to achieve the intended purpose. More specifically, the composition preferably comprises a therapeutically effective amount of therapeutically active agent, that is, an amount of therapeutically active agent effective to prevent, alleviate or ameliorate symptoms of a disorder or prolong the survival of the subject being treated. Furthermore, an amount of absorption enhancer is preferably effective for enhancing absorption of the therapeutically active agent (e.g., in a manner described herein); and an amount of protease inhibitor (if present) is preferably effective for inhibiting degradation of the therapeutically active agent (e.g., a polypeptide agent) by a protease.

Determination of a therapeutically effective amount is well within the capability of those skilled in the art, especially in light of the detailed disclosure provided herein.

For any preparation used in the methods of the invention, the therapeutically effective amount or dose can be estimated initially from *in vitro* and cell culture assays. For example, a dose can be formulated in animal models to achieve a desired concentration or titer. Such information can be used to more accurately determine useful doses in humans.

Toxicity and therapeutic efficacy of the therapeutically active agent described herein can be determined by standard pharmaceutical procedures *in vitro,* in cell cultures or experimental animals. The data obtained from these *in vitro* and cell culture assays and animal studies can be used in formulating a range of dosage for use in human. The dosage may vary depending upon the dosage form employed and the route of administration utilized. The exact formulation and dosage can be chosen by the individual physician in view of the patient's condition. (See e.g., Fingl et al., 1975, in "The Pharmacological Basis of Therapeutics", Ch. 1 p.1).

Dosage amount and interval may be adjusted individually to provide levels (e.g., plasma levels) of the therapeutically active agent sufficient to induce or suppress a biological effect (minimal effective concentration, MEC). The MEC will vary for each preparation, but can be estimated from *in vitro* data. Dosages necessary to achieve the MEC will depend on individual characteristics. Detection assays can be used to determine plasma concentrations.

In some of any of the embodiments described herein, a dosage of the therapeutically active agent according to any of the respective embodiments described herein (e.g., an amount of the active agent in a unit dosage form composition, according to any of the respective embodiments described herein) is at least 50 µg of therapeutically active agent. In some embodiments, the dosage is at least 100 µg of therapeutically active agent. In some embodiments, the dosage is at least 200 µg of therapeutically active agent. In some embodiments, the dosage is at least 500 µg of therapeutically active agent. In some embodiments, the dosage is at least 1000 µg of therapeutically active agent. In some of the aforementioned embodiments, the absorption enhancer is NAC (8-N-(2-hydroxybenzoyl)aminocaprylic acid) or a salt thereof (e.g., sodium 8-N-(2-hydroxybenzoyl)aminocaprylate).

In some of any of the embodiments described herein, a dosage of the therapeutically active agent (e.g., an amount of the active agent in a unit dosage form composition, according to any of the respective embodiments described herein) is 3000 µg or less (e.g., from 50 to 3000 µg or from 100 to 3000 µg or from 200 to 3000 µg or from 500 to 3000 µg or from 1000 to 3000 µg) of therapeutically active agent. In some embodiments, the dosage is 2000 µg or less (e.g., from 50 to 2000 µg or from 100 to 2000 µg or from 200 to 2000 µg or from 500 to 2000 µg or from 1000 to 2000 µg) of therapeutically active agent. In some embodiments, the dosage is 1000 µg or less (e.g., from 50 to 1000 µg or from 100 to 1000 µg or from 200 to 1000 µg or from 500 to 1000 µg) of therapeutically active agent. In some of the aforementioned embodiments, the absorption enhancer is NAC (8-N-(2-hydroxybenzoyl)aminocaprylic acid) or a salt thereof (e.g., sodium 8-N-(2-hydroxybenzoyl)aminocaprylate).

Depending on the severity and responsiveness of the condition to be treated, dosing can be of a single or a plurality of administrations, with course of treatment lasting from several hours to several weeks or until cure is effected or diminution of the disease state is achieved.

The amount of a composition to be administered will, of course, be dependent on the subject being treated, the severity of the affliction, the manner of administration, the judgment of the prescribing physician, etc.

Each dosing may optionally be effected using a single dosage form, or a plurality of unit dosage forms. A plurality of unit dosage forms may optionally be used merely for convenience (e.g., wherein two or three unit dosage forms are used), or alternatively, to reduce variability in absorption according to any of the respective embodiments described in International Patent Application Publication No. WO 2018/033927, for example, wherein at least three or at least four unit dosage forms are used (e.g., from 3 to 10 or from 4 to 10, including any intermediate values and subranges therebetween).

Compositions of some embodiments of the invention may, if desired, be presented in a pack or dispenser device, such as an FDA approved kit, which may contain one or more unit dosage forms containing the active ingredient. The pack may, for example, comprise metal or plastic foil, such as a blister pack. The pack or dispenser device may be accompanied by instructions for administration. The pack or dispenser may also be accommodated by a notice associated with the container in a form prescribed by a governmental agency regulating the manufacture, use or sale of pharmaceuticals, which notice is reflective of approval by the agency of the form of the compositions or human or veterinary administration. Such notice, for example, may be of labeling approved by the U.S. Food and Drug Administration for prescription drugs or of an approved product insert. Compositions comprising a preparation of the invention may also be prepared (e.g., as described herein), placed in an appropriate container, and labeled for treatment of an indicated condition, as is further detailed herein.

In some embodiments of any one of the embodiments described herein, when the therapeutically active agents is a polypeptide, the composition further comprises at least one protease inhibitor, for example, a type of protease inhibitor, a concentration and/or absolute amount of protease inhibitor, and/or a ratio of protease inhibitor to absorption enhancer and/or therapeutically active agent according to any one of the embodiments relating to a protease inhibitor described in any of U.S. Patent Application Publication No. 2011/0142800, and International Patent Application Publications WO 2016/128972 and WO 2018/033927.

Herein throughout, the term "protease inhibitor" refers to a compound which reduces a proteolytic activity of a protease, for example, a proteolytic activity which inactivates a therapeutically active agent described herein. The term "protease inhibitor" encompasses, for example, both large molecules (e.g., proteins) and small molecules, as well as both naturally occurring compounds and synthetic compounds.

In some embodiments of any of the embodiments described herein, the at least one protease inhibitor comprises at least one trypsin inhibitor. In some embodiments, the at least one protease inhibitor consists essentially of one or more trypsin inhibitor(s).

Examples of trypsin inhibitors which may be utilized in any one of the embodiments described herein include, without limitation, lima bean trypsin inhibitor, aprotinin, soybean trypsin inhibitor, ovomucoid trypsin inhibitor and any combination thereof. In some embodiments, the at least one trypsin inhibitor comprises soybean trypsin inhibitor (SBTI). In some embodiments, the at least one trypsin inhibitor (an optionally the at least one protease inhibitor) consists essentially of SBTI.

In some embodiments of any of the embodiments described herein, the at least one protease inhibitor comprises at least one serpin. In some embodiments, the at least one protease inhibitor consists essentially of one or more serpin(s).

Examples of serpins which may be utilized in any one of the embodiments described herein, include, without limitation, alpha 1-antitrypsin, antitrypsin-related protein, alpha 1-antichymotrypsin, kallistatin, protein C inhibitor, cortisol binding globulin, thyroxine-binding globulin, angiotensinogen, centerin, protein Z-related protease inhibitor, vaspin, monocyte/neutrophil elastase inhibitor, plasminogen activator inhibitor-2, squamous cell carcinoma antigen-1 (SCCA-1), squamous cell carcinoma antigen-2 (SCCA-2), maspin, proteinase inhibitor 6 (PI-6), megsin, serpin B8 (PI-8), serpin B9 (PI-9), bomapin, yukopin, hurpin/headpin, antithrombin, heparin cofactor II, plasminogen activator inhibitor 1, glia-derived nexin, pigment epithelium derived factor, alpha 2-antiplasmin, complement 1-inhibitor, 47 kDa heat shock protein (HSP47), neuroserpin and pancpin.

In some embodiments of any of the embodiments described herein, the at least one protease inhibitor comprises at least one cysteine protease inhibitor. In some embodiments, the at least one protease inhibitor consists essentially of one or more cysteine protease inhibitor(s).

Examples of cysteine protease inhibitors which may be utilized in any one of the embodiments described herein include, without limitation, type 1 cystatins, type 2 cystatins, human cystatins C, D, S, SN, and SA, cystatin E/M, cystatin F, and type 3 cystatins (including kininogens).

In some embodiments of any of the embodiments described herein, the at least one protease inhibitor comprises at least one threonine protease inhibitor. In some embodiments, the at least one protease inhibitor consists essentially of one or more threonine protease inhibitor(s).

Examples of threonine protease inhibitors which may be utilized in any one of the embodiments described herein include, without limitation, bortezomib, MLN-519, ER-807446 and TMC-95A.

In some embodiments of any of the embodiments described herein, the at least one protease inhibitor comprises at least one aspartic protease inhibitor. In some embodiments, the at least one protease inhibitor consists essentially of one or more aspartic protease inhibitor(s).

Examples of aspartic protease inhibitors which may be utilized in any one of the embodiments described herein, include, without limitation, α₂-macroglobulin, pepstatin A, aspartic protease inhibitor 11, aspartic protease inhibitor 1, aspartic protease inhibitor 2, aspartic protease inhibitor 3, aspartic protease inhibitor 4, aspartic protease inhibitor 5, aspartic protease inhibitor 6, aspartic protease inhibitor 7, aspartic protease inhibitor 8, aspartic protease inhibitor 9, pepsin inhibitor Dit33, and protease A inhibitor 3.

In some embodiments of any of the embodiments described herein, the at least one protease inhibitor comprises at least one metalloprotease inhibitor. In some embodiments, the at least one protease inhibitor consists essentially of one or more metalloprotease inhibitor(s).

Examples of metalloprotease inhibitors which may be utilized in any one of the embodiments described herein, include, without limitation, angiotensin-1-converting enzyme inhibitory peptide, antihemorrhagic factor BJ46a, beta-casein, proteinase inhibitor CeKI, venom metalloproteinase inhibitor DM43, carboxypeptidase A inhibitor, smpI, IMPI, alkaline proteinase, latexin, carboxypeptidase inhibitor, antihemorrhagic factor HSF, testican-3, SPOCK3, TIMP1, metalloproteinase inhibitor 1, metalloproteinase inhibitor 2, TIMP2, metalloproteinase inhibitor 3, TIMP3, metalloproteinase inhibitor 4, TIMP4, putative metalloproteinase inhibitor tag-225, tissue inhibitor of metalloprotease, WAP, kazal inhibitor, immunoglobulin, and kunitz and NTR domain-containing protein 1.

Examples of protease inhibitors which may be utilized in any one of the embodiments described herein also include, without limitation, AEBSF-HCl, ε-aminocaproic acid, α1-antichymotypsin, antipain, antithrombin III, α1-antitrypsin, APMSF (4-amidinophenyl-methane sulfonyl-fluoride), sprotinin, benzamidine, chymostatin, DFP (diisopropylfluoro-phosphate), leupeptin, 4-(2-Aminoethyl)-benzenesulfonyl fluoride hydrochloride, PMSF (phenylmethyl sulfonyl fluoride), TLCK (1-chloro-3-tosylamido-7-amino-2-heptanone), TPCK (1-chloro-3-tosylamido-4-phenyl-2-butanone), pentamidine isothionate, pepstatin, guanidium, α2-macroglobulin, a chelating agent of zinc, and iodoacetate.

In some embodiments of any one of the embodiments described herein, the amount of a protease inhibitor in a unit dosage form described herein is at least about 0.1 mg. In some embodiments, the amount of a protease inhibitor in a unit dosage form described herein is at least about 0.2 mg. In some embodiments, the amount of a protease inhibitor in a unit dosage form described herein is at least about 0.3 mg. In some embodiments, the amount of a protease inhibitor in a unit dosage form described herein is at least about 0.4 mg. In some embodiments, the amount of a protease inhibitor in a unit dosage form described herein is at least about 0.6 mg. In some embodiments, the amount of a protease inhibitor in a unit dosage form described herein is at least about 0.8 mg. In some embodiments, the amount of a protease inhibitor in a unit dosage form described herein is at least about 1 mg. In some embodiments, the amount of a protease inhibitor in a unit dosage form described herein is at least about 1.5 mg. In some embodiments, the amount of a protease inhibitor in a unit dosage form described herein is at least about 2 mg. In some embodiments, the amount of a protease inhibitor in a unit dosage form described herein is at least about 2.5 mg. In some embodiments, the amount of a protease inhibitor in a unit dosage form described herein is at least about 3 mg. In some embodiments, the amount of a protease inhibitor in a unit dosage form described herein is at least about 5 mg. In some embodiments, the amount of a protease inhibitor in a unit dosage form described herein is at least about 7 mg. In some embodiments, the amount of a protease inhibitor in a unit dosage form described herein is at least about 10 mg. In some embodiments, the amount of a protease inhibitor in a unit dosage form described herein is at least about 12 mg. In some embodiments, the amount of a protease inhibitor in a unit dosage form described herein is at least about 15 mg. In some embodiments, the amount of a protease inhibitor in a unit dosage form described herein is at least about 20 mg. In some embodiments, the amount of a protease inhibitor in a unit dosage form described herein is at least about 30 mg. In some embodiments, the amount of a protease inhibitor in a unit dosage form described herein is at least about 50 mg. In some embodiments, the amount of a protease inhibitor in a unit dosage form described herein is at least about 70 mg. In some embodiments, the amount of a protease inhibitor in a unit dosage form described herein is at least about 100 mg.

In some embodiments of any one of the embodiments described herein, the amount of a protease inhibitor in a unit dosage form described herein is in a range of from 0.1 to 1 mg. In some embodiments, the amount of a protease inhibitor in a unit dosage form described herein is in a range of from 0.2 to 1 mg. In some embodiments, the amount of a protease inhibitor in a unit dosage form described herein is in a range of from 0.3 to 1 mg. In some embodiments, the amount of a protease inhibitor in a unit dosage form described herein is in a range of from 0.5 to 1 mg.

In some embodiments of any one of the embodiments described herein, the amount of a protease inhibitor in a unit dosage form described herein is in a range of from 0.1 to 2 mg. In some embodiments, the amount of a protease inhibitor in a unit dosage form described herein is in a range of from 0.2 to 2 mg. In some embodiments, the amount of a protease inhibitor in a unit dosage form described herein is in a range of from 0.3 to 2 mg. In some embodiments, the amount of a protease inhibitor in a unit dosage form described herein is in a range of from 0.5 to 2 mg. In some embodiments, the amount of a protease inhibitor in a unit dosage form described herein is in a range of from 1 to 2 mg.

In some embodiments of any one of the embodiments described herein, the amount of a protease inhibitor in a unit dosage form described herein is in a range of from 1 to 10 mg. In some embodiments, the amount of a protease inhibitor in a unit dosage form described herein is in a range of from 2 to 10 mg. In some embodiments, the amount of a protease inhibitor in a unit dosage form described herein is in a range of from 3 to 10 mg. In some embodiments, the amount of a protease inhibitor in a unit dosage form described herein is in a range of from 5 to 10 mg.

In some embodiments of any one of the embodiments described herein, the amount of a protease inhibitor in a unit dosage form described herein is in a range of from 1 to 20 mg. In some embodiments, the amount of a protease inhibitor in a unit dosage form described herein is in a range of from 2 to 20 mg. In some embodiments, the amount of a protease inhibitor in a unit dosage form described herein is in a range of from 3 to 20 mg. In some embodiments, the amount of a protease inhibitor in a unit dosage form described herein is in a range of from 5 to 20 mg. In some embodiments, the amount of a protease inhibitor in a unit dosage form described herein is in a range of from 10 to 20 mg.

In some embodiments of any one of the embodiments described herein, the amount of a protease inhibitor in a unit dosage form described herein is in a range of from 10 to 100 mg. In some embodiments, the amount of a protease inhibitor in a unit dosage form described herein is in a range of from 20 to 100 mg. In some embodiments, the amount of a protease inhibitor in a unit dosage form described herein is in a range of from 30 to 100 mg. In some embodiments, the amount of a protease inhibitor in a unit dosage form described herein is in a range of from 50 to 100 mg.

In some embodiments of any one of the embodiments described herein, the amount of a protease inhibitor in a unit dosage form described herein is in a range of from 10 to 200 mg. In some embodiments, the amount of a protease inhibitor in a unit dosage form described herein is in a range of from 20 to 200 mg. In some embodiments, the amount of a protease inhibitor in a unit dosage form described herein is in a range of from 30 to 200 mg. In some embodiments, the amount of a protease inhibitor in a unit dosage form described herein is in a range of from 50 to 200 mg. In some embodiments, the amount of a protease inhibitor in a unit dosage form described herein is in a range of from 100 to 200 mg.

In some embodiments of any one of the embodiments described herein, the amount of a protease inhibitor in a unit dosage form described herein is at least about 10 kallikrein inactivator units (k.i.u.). In some embodiments, the amount of a protease inhibitor in a unit dosage form described herein is at least about 12 k.i.u. In some embodiments, the amount of a protease inhibitor in a unit dosage form described herein is at least about 15 k.i.u. In some embodiments, the amount of a protease inhibitor in a unit dosage form described herein is at least about 20 k.i.u. In some embodiments, the amount of a protease inhibitor in a unit dosage form described herein is at least about 30 k.i.u. In some embodiments, the amount of a protease inhibitor in a unit dosage form described herein is at least about 40 k.i.u. In some embodiments, the amount of a protease inhibitor in a unit dosage form described herein is at least about 50 k.i.u. In some embodiments, the amount of a protease inhibitor in a unit dosage form described herein is at least about 70 k.i.u. In some embodiments, the amount of a protease inhibitor in a unit dosage form described herein is at least about 100 k.i.u. In some embodiments, the amount of a protease inhibitor in a unit dosage form described herein is at least about 150 k.i.u. In some embodiments, the amount of a protease inhibitor in a unit dosage form described herein is at least about 200 k.i.u. In some embodiments, the amount of a protease inhibitor in a unit dosage form described herein is at least about 300 k.i.u. In some embodiments, the amount of a protease inhibitor in a unit dosage form described herein is at least about 500 k.i.u. In some embodiments, the amount of a protease inhibitor in a unit dosage form described herein is at least about 700 k.i.u. In some embodiments, the amount of a protease inhibitor in a unit dosage form described herein is at least about 1000 k.i.u. In some embodiments, the amount of a protease inhibitor in a unit dosage form described herein is at least about 1500 k.i.u. In some embodiments, the amount of a protease inhibitor in a unit dosage form described herein is at least about 3000 k.i.u. In some embodiments, the amount of a protease inhibitor in a unit dosage form described herein is at least about 4000 k.i.u. In some embodiments, the amount of a protease inhibitor in a unit dosage form described herein is at least about 5000 k.i.u.

Herein and in the art, a "kallikrein inactivating unit" (k.i.u) refers to an amount of protease inhibitor that has the ability to inhibit 2 units of kallikrein by 50 % (e.g., in aqueous solution at an optimal pH and solution volume for activity of the protease inhibitor).

In some embodiments of any one of the embodiments described herein, a weight ratio of protease inhibitor to therapeutically active agent is in a range of from 1:1 to 5:1 (protease inhibitor: therapeutically active agent). In some embodiments, a weight ratio of protease inhibitor to therapeutically active agent is in a range of from 5:1 to 10:1. In some embodiments, a weight ratio of protease inhibitor to therapeutically active agent is in a range of from 10:1 to 20:1. In some embodiments, a weight ratio of protease inhibitor to therapeutically active agent is in a range of from 20:1 to 30:1. In some embodiments, a weight ratio of protease inhibitor to therapeutically active agent is in a range of from 30:1 to 40:1. In some embodiments, a weight ratio of protease inhibitor to therapeutically active agent is in a range of from 40:1 to 50:1. In some embodiments, a weight ratio of protease inhibitor to therapeutically active agent is in a range of from 50:1 to 75:1. In some embodiments, a weight ratio of protease inhibitor to therapeutically active agent is in a range of from 75:1 to 100:1. In some embodiments, a weight ratio of protease inhibitor to therapeutically active agent is in a range of from 100:1 to 200:1. In some embodiments, a weight ratio of protease inhibitor to therapeutically active agent is in a range of from 200:1 to 300:1. In some embodiments, a weight ratio of protease inhibitor to therapeutically active agent is in a range of from 300:1 to 400:1. In some embodiments, a weight ratio of protease inhibitor to therapeutically active agent is in a range of from 400:1 to 500:1. In some embodiments, the protease inhibitor is soybean trypsin inhibitor.

### Additional definitions:

Herein, the term "polypeptide" refers to a polymer comprising at least 4 amino acid residues linked by peptide bonds or analogs thereof (as described herein), and optionally only by peptide bonds *per se.* In some embodiments, the polypeptide comprises at least 10 amino acid residues or analogs thereof. In some embodiments, the polypeptide comprises at least 20 amino acid residues or analogs thereof. In some embodiments, the polypeptide comprises at least 30 amino acid residues or analogs thereof. In some embodiments, the polypeptide comprises at least 50 amino acid residues or analogs thereof. The term "polypeptide" encompasses native polypeptides (e.g., degradation products, synthetically synthesized polypeptides and/or recombinant polypeptides), including, without limitation, native proteins, fragments and substituted derivatives of native proteins and homologs of native proteins and/or fragments and/or substituted derivatives thereof; as well as peptidomimetics (typically, synthetically synthesized polypeptides) and peptoids and semipeptoids which are polypeptide analogs, which may have, for example, modifications rendering the polypeptides more stable while in a body or more capable of penetrating into cells. Such modifications include, but are not limited to N-terminus modification, C-terminus modification, peptide bond modification, backbone modifications, and residue modification. Methods for preparing peptidomimetic compounds are well known in the art and are specified, for example, in Quantitative Drug Design, C.A. Ramsden Gd., Chapter 17.2, F. Choplin Pergamon Press (1992). Further details in this respect are provided herein.

Peptide bonds (-CO-NH-) within the polypeptide may be substituted, for example, by N-methylated amide bonds (-N(CH₃)-CO-), ester bonds (-C(=O)-O-), ketomethylene bonds (-CO-CH₂-), sulfinylmethylene bonds (-S(=O)-CH₂-), α-aza bonds (-NH-N(R)-CO-), wherein R is any alkyl (e.g., methyl), amine bonds (-CH₂-NH-), sulfide bonds (-CH₂-S-), ethylene bonds (-CH₂-CH₂-), hydroxyethylene bonds (-CH(OH)-CH₂-), thioamide bonds (-CS-NH-), olefinic double bonds (-CH=CH-), fluorinated olefinic double bonds (-CF=CH-), retro amide bonds (-NH-CO-), peptide derivatives (-N(R)-CH2-CO-), wherein R is the "normal" side chain, naturally present on the carbon atom.

These modifications can occur at any of the bonds along the polypeptide chain and even at several (2-3) bonds at the same time.

Natural aromatic amino acids, Trp, Tyr and Phe, may be substituted by non-natural aromatic amino acids such as 1,2,3,4-tetrahydroisoquinoline-3-carboxylic acid (Tic), naphthylalanine, ring-methylated derivatives of Phe, halogenated derivatives of Phe or O-methyl-Tyr.

The polypeptides of some embodiments of the invention (e.g., a therapeutically active agent described herein) may also include one or more modified amino acids or one or more non-amino acid monomers (e.g. fatty acids, complex carbohydrates etc.).

The term "amino acid" or "amino acids" is understood to include the 20 naturally occurring amino acids; those amino acids often modified post-translationally *in vivo,* including, for example, hydroxyproline, phosphoserine and phosphothreonine; and other unusual amino acids including, but not limited to, 2-aminoadipic acid, hydroxylysine, isodesmosine, nor-valine, nor-leucine and ornithine. Furthermore, the term "amino acid" includes both D- and L-amino acids.

Tables A and B below list naturally occurring amino acids (Table A), and non-conventional or modified amino acids (e.g., synthetic, Table B) which can be used with some embodiments of the invention.

**Table A**

| ***Amino Acid*** | ***Three-Letter Abbreviation*** | ***One-letter Symbol*** |
|---|---|---|
| Alanine | Ala | A |
| Arginine | Arg | R |
| Asparagine | Asn | N |
| Aspartic acid | Asp | D |
| Cysteine | Cys | C |
| Glutamine | Gln | Q |
| Glutamic Acid | Glu | E |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |
| Any amino acid as above | Xaa | X |

**Table B**

| ***Non-conventional amino acid*** | ***Code*** | ***Non-conventional amino acid*** | ***Code*** |
|---|---|---|---|
| ornithine | Orn | hydroxyproline | Hyp |
| α-aminobutyric acid | Abu | aminonorbornyl-carboxylate | Norb |
| D-alanine | Dala | aminocyclopropane-carboxylate | Cpro |
| D-arginine | Darg | N-(3-guanidinopropyl) glycine | Narg |
| D-asparagine | Dasn | N-(carbamylmethyl)glycine | Nasn |
| D-aspartic acid | Dasp | N-(carboxymethyl)glycine | Nasp |
| D-cysteine | Dcys | N-(thiomethyl)glycine | Ncys |
| D-glutamine | Dgln | N-(2-carbamylethyl)glycine | Ngln |
| D-glutamic acid | Dglu | N-(2-carboxyethyl)glycine | Nglu |
| D-histidine | Dhis | N-(imidazolylethyl)glycine | Nhis |
| D-isoleucine | Dile | N-(1-methylpropyl)glycine | Nile |
| D-leucine | Dleu | N-(2-methylpropyl)glycine | Nleu |
| D-lysine | Dlys | N-(4-aminobutyl)glycine | Nlys |
| D-methionine | Dmet | N-(2-methylthioethyl)glycine | Nmet |
| D-ornithine | Dorn | N-(3-aminopropyl)glycine | Norn |
| D-phenylalanine | Dphe | N-benzylglycine | Nphe |
| D-proline | Dpro | N-(hydroxymethyl) glycine | Nser |
| D-serine | Dser | N-(1-hydroxyethyl)glycine | Nthr |
| D-threonine | Dthr | N-(3-indolylethyl) glycine | Nhtrp |
| D-tryptophan | Dtrp | N-(*p*-hydroxyphenyl)glycine | Ntyr |
| D-tyrosine | Dtyr | N-(1-methylethyl) glycine | Nval |
| D-valine | Dval | N-methylglycine | Nmgly |
| D-N-methylalanine | Dnmala | L-N-methylalanine | Nmala |
| D-N-methylarginine | Dnmarg | L-N-methylarginine | Nmarg |
| D-N-methylasparagine | Dnmasn | L-N-methylasparagine | Nmasn |
| D-N-methylasparatate | Dnmasp | L-N-methylaspartic acid | Nmasp |
| D-N-methylcysteine | Dnmcys | L-N-methylcysteine | Nmcys |
| D-N-methylglutamine | Dnmgln | L-N-methylglutamine | Nmgln |
| D-N-methylglutamate | Dnmglu | L-N-methylglutamic acid | Nmglu |
| D-N-methylhistidine | Dnmhis | L-N-methylhistidine | Nmhis |
| D-N-methylisoleucine | Dnmile | L-N-methylisolleucine | Nmile |
| D-N-methylleucine | Dnmleu | L-N-methylleucine | Nmleu |
| D-N-methyllysine | Dnmlys | L-N-methyllysine | Nmlys |
| D-N-methylmethionine | Dnmmet | L-N-methylmethionine | Nmmet |
| D-N-methylornithine | Dnmorn | L-N-methylornithine | Nmorn |
| D-N-methylphenylalanine | Dnmphe | L-N-methylphenylalanine | Nmphe |
| D-N-methylproline | Dnmpro | L-N-methylproline | Nmpro |
| D-N-methylserine | Dnmser | L-N-methylserine | Nmser |
| D-N-methylthreonine | Dnmthr | L-N-methylthreonine | Nmthr |
| D-N-methyltryptophan | Dnmtrp | L-N-methyltryptophan | Nmtrp |
| D-N-methyltyrosine | Dnmtyr | L-N-methyltyrosine | Nmtyr |
| D-N-methylvaline | Dnmval | L-N-methylvaline | Nmval |
| L-norleucine | Nle | L-N-methylnorleucine | Nmnle |
| L-norvaline | Nva | L-N-methylnorvaline | Nmnva |
| L-ethylglycine | Etg | L-N-methyl-ethylglycine | Nmetg |
| L-t-butylglycine | Tbug | L-N-methyl-t-butylglycine | Nmtbug |
| L-homophenylalanine | Hphe | L-N-methyl-homophenylalanine | Nmhphe |
| α-naphthylalanine | Anap | N-methyl-α-naphthylalanine | Nmanap |
| penicillamine | Pen | N-methylpenicillamine | Nmpen |
| γ-aminobutyric acid | Gabu | N-methyl-γ-aminobutyrate | Nmgabu |
| cyclohexylalanine | Chexa | N-methyl-cyclohexylalanine | Nmchexa |
| cyclopentylalanine | Cpen | N-methyl-cyclopentylalanine | Nmcpen |
| α-amino-α-methylbutyrate | Aabu | N-methyl-α-amino-α-methylbutyrate | Nmaabu |
| α-aminoisobutyric acid | Aib | N-methyl-α-aminoisobutyrate | Nmaib |
| D-α-methylarginine | Dmarg | L-α-methylarginine | Marg |
| D-α-methylasparagine | Dmasn | L-α-methylasparagine | Masn |
| D-α-methylaspartate | Dmasp | L-α-methylaspartate | Masp |
| D-α-methylcysteine | Dmcys | L-α-methylcysteine | Mcys |
| D-α-methylglutamine | Dmgln | L-α-methylglutamine | Mgln |
| D-α-methyl glutamic acid | Dmglu | L-α-methylglutamate | Mglu |
| D-α-methylhistidine | Dmhis | L-α-methylhistidine | Mhis |
| D-α-methylisoleucine | Dmile | L-α-methylisoleucine | Mile |
| D-α-methylleucine | Dmleu | L-α-methylleucine | Mleu |
| D-α-methyllysine | Dmlys | L-α-methyllysine | Mlys |
| D-α-methylmethionine | Dmmet | L-α-methylmethionine | Mmet |
| D-α-methylornithine | Dmorn | L-α-methylornithine | Morn |
| D-α-methylphenylalanine | Dmphe | L-α-methylphenylalanine | Mphe |
| D-α-methylproline | Dmpro | L-α-methylproline | Mpro |
| D-α-methylserine | Dmser | L-α-methylserine | Mser |
| D-α-methylthreonine | Dmthr | L-α-methylthreonine | Mthr |
| D-α-methyltryptophan | Dmtrp | L-α-methyltryptophan | Mtrp |
| D-α-methyltyrosine | Dmtyr | L-α-methyltyrosine | Mtyr |
| D-α-methylvaline | Dmval | L-α-methylvaline | Mval |
| N-cyclobutylglycine | Ncbut | L-α-methylnorvaline | Mnva |
| N-cycloheptylglycine | Nchep | L-α-methylethylglycine | Metg |
| N-cyclohexylglycine | Nchex | L-α-methyl-t-butylglycine | Mtbug |
| N-cyclodecylglycine | Ncdec | L-α-methyl-homophenylalanine | Mhphe |
| N-cyclododecylglycine | Ncdod | α-methyl-α-naphthylalanine | Manap |
| N-cyclooctylglycine | Ncoct | α-methylpenicillamine | Mpen |
| N-cyclopropylglycine | Ncpro | α-methyl-γ-aminobutyrate | Mgabu |
| N-cycloundecylglycine | Ncund | α-methyl-cyclohexylalanine | Mchexa |
| N-(2-aminoethyl)glycine | Naeg | α-methyl-cyclopentylalanine | Mcpen |
| N-(2,2-diphenylethyl)glycine | Nbhm | N-(N-(2,2-diphenylethyl) carbamylmethyl-glycine | Nnbhm |
| N-(3,3-diphenylpropyl)glycine | Nbhe | N-(N-(3,3-diphenylpropyl) carbamylmethyl-glycine | Nnbhe |
| 1-carboxy-1-(2,2-diphenyl ethylamino)cyclopropane | Nmbc | 1,2,3,4-tetrahydroisoquinoline-3-carboxylic acid | Tic |
| phosphoserine | pSer | phosphothreonine | pThr |
| phosphotyrosine | pTyr | O-methyl-tyrosine | |
| 2-aminoadipic acid | | hydroxylysine | |

***(Table B Cont.)***

The polypeptides of some embodiments of the invention (e.g., a therapeutically active agent described herein) are preferably utilized in a linear form, although it will be appreciated that in cases where cyclization does not severely interfere with polypeptide characteristics, cyclic forms of the polypeptide can also be utilized.

In some embodiments of any one of the embodiments described herein, the polypeptide is water-soluble, as defined herein.

Water-soluble polypeptides preferably include one or more non-natural or natural polar amino acids, including but not limited to serine and threonine which are capable of increasing polypeptide water-solubility due to their hydroxyl-containing side chain. Optionally, a homolog of a polypeptide is selected so as to be more water-soluble than the parent polypeptide, for example, by replacing one or more amino acids in the polypeptide with polar amino acids.

The polypeptides of some embodiments of the invention (e.g., a therapeutically active agent described herein) may be synthesized by any techniques that are known to those skilled in the art of peptide synthesis. For solid phase peptide synthesis, a summary of the many techniques may be found in J. M. Stewart and J. D. Young, Solid Phase Peptide Synthesis, W. H. Freeman Co. (San Francisco), 1963 and J. Meienhofer, Hormonal Proteins and Peptides, vol. 2, p. 46, Academic Press (New York), 1973. For classical solution synthesis see G. Schroder and K. Lupke, The Peptides, vol. 1, Academic Press (New York), 1965.

In general, these methods comprise the sequential addition of one or more amino acids or suitably protected amino acids to a growing polypeptide chain. Normally, either the amino or carboxyl group of the first amino acid is protected by a suitable protecting group. The protected or derivatized amino acid can then either be attached to an inert solid support or utilized in solution by adding the next amino acid in the sequence having the complimentary (amino or carboxyl) group suitably protected, under conditions suitable for forming the amide linkage. The protecting group is then removed from this newly added amino acid residue and the next amino acid (suitably protected) is then added, and so forth. After all the desired amino acids have been linked in the proper sequence, any remaining protecting groups (and any solid support) are removed sequentially or concurrently, to afford the final polypeptide compound. By simple modification of this general procedure, it is possible to add more than one amino acid at a time to a growing chain, for example, by coupling (under conditions which do not racemize chiral centers) a protected tripeptide with a properly protected dipeptide to form, after deprotection, a pentapeptide and so forth. Further description of peptide synthesis is disclosed in U.S. Pat. No. 6,472,505.

A preferred method of preparing the polypeptide compounds of some embodiments of the invention (e.g., a therapeutically active agent described herein) involves solid phase peptide synthesis.

Large scale polypeptide synthesis is described by Andersson et al. [Biopolymers 2000; 55:227-250].

Herein, a "homolog" of a given polypeptide refers to a polypeptide that exhibits at least 80 % homology, preferably at least 90 % homology, and more preferably at least 95 % homology, and more preferably at least 98 % homology to the given polypeptide. In some embodiments, a homolog of a given polypeptide further shares a therapeutic activity with the given polypeptide. The percentage of homology refers to the percentage of amino acid residues in a first polypeptide sequence which match a corresponding residue of a second polypeptide sequence to which the first polypeptide is being compared. Generally, the polypeptides are aligned to give maximum homology. A variety of strategies are known in the art for performing comparisons of amino acid sequences in order to assess degrees of identity, including, for example, manual alignment, computer assisted sequence alignment and combinations thereof. A number of algorithms (which are generally computer implemented) for performing sequence alignment are widely available, or can be produced by one of skill in the art. Representative algorithms include, e.g., the local homology algorithm of Smith and Waterman (Adv. Appl. Math., 1981, 2: 482); the homology alignment algorithm of Needleman and Wunsch (J. Mol. Biol., 1970, 48: 443); the search for similarity method of Pearson and Lipman (Proc. Natl. Acad. Sci. (USA), 1988, 85: 2444); and/or by computerized implementations of these algorithms (e.g., GAP, BESTFIT, FASTA, and TFASTA in the Wisconsin Genetics Software Package Release 7.0, Genetics Computer Group, 575 Science Dr., Madison, Wis.). Readily available computer programs incorporating such algorithms include, for example, BLASTN, BLASTP, Gapped BLAST, PILEUP, CLUSTALW etc. When utilizing BLAST and Gapped BLAST programs, default parameters of the respective programs may be used. Alternatively, the practitioner may use non-default parameters depending on his or her experimental and/or other requirements (see for example, the Web site having URL www(dot)ncbi(dot)nlm(dot)nih(dot)gov).

Herein, the terms "amine" and "amino" each refer to a -NR'R" group, wherein R' and R" are each hydrogen or a substituted or non-substituted alkyl, alkenyl, alkynyl, cycloalkyl, heteroalicyclic (linked to amine nitrogen via a ring carbon thereof), aryl, or heteroaryl (linked to amine nitrogen via a ring carbon thereof), as defined herein. Alternatively, R' and R" may optionally be linked to form a heteroalicyclic ring (as defined herein). Optionally, R' and R" and R‴ are hydrogen or alkyl comprising 1 to 4 carbon atoms. Optionally, at least one of R' and R" is hydrogen and optionally both are hydrogen. When substituted, the carbon atom of an R' and R" hydrocarbon moiety which is bound to the nitrogen atom of the amine is not substituted by oxo, such that R' and R" are not (for example) carbonyl, C-carboxy or amide, as these groups are defined herein.

As used herein throughout, the term "alkyl" refers to any saturated aliphatic hydrocarbon including straight chain and branched chain groups. Preferably, the alkyl group has 1 to 20 carbon atoms. Whenever a numerical range; e.g., "1 to 20", is stated herein, it implies that the group, in this case the hydrocarbon, may contain 1 carbon atom, 2 carbon atoms, 3 carbon atoms, etc., up to and including 20 carbon atoms. More preferably, the alkyl is a medium size alkyl having 1 to 10 carbon atoms. Most preferably, unless otherwise indicated, the alkyl is a lower alkyl having 1 to 4 carbon atoms. The alkyl group may be substituted or non-substituted. When substituted, the substituent group can be, for example, cycloalkyl, aryl, heteroaryl, heteroalicyclic, halo, hydroxy, alkoxy, aryloxy, thiohydroxy, thioalkoxy, thioaryloxy, sulfinyl, sulfonyl, sulfonate, sulfate, cyano, nitro, azide, phosphonyl, phosphinyl, oxo, imine, oxime, hydrazone, carbonyl, thiocarbonyl, a urea group, a thiourea group, O-carbamyl, N-carbamyl, O-thiocarbamyl, N-thiocarbamyl, S-thiocarbamyl, C-amido, N-amido, C-carboxy, O-carboxy, sulfonamido, guanyl, guanidinyl, hydrazine, hydrazide, thiohydrazide, and amino, as these terms are defined herein.

Herein, the term "alkenyl" describes an unsaturated aliphatic hydrocarbon comprise at least one carbon-carbon double bond, including straight chain and branched chain groups. Preferably, the alkenyl group has 2 to 20 carbon atoms. More preferably, the alkenyl is a medium size alkenyl having 2 to 10 carbon atoms. Most preferably, unless otherwise indicated, the alkenyl is a lower alkenyl having 2 to 4 carbon atoms. The alkenyl group may be substituted or non-substituted. Substituted alkenyl may have one or more substituents, whereby each substituent group can independently be, for example, alkynyl, cycloalkyl, alkynyl, aryl, heteroaryl, heteroalicyclic, halo, hydroxy, alkoxy, aryloxy, thiohydroxy, thioalkoxy, thioaryloxy, sulfinyl, sulfonyl, sulfonate, sulfate, cyano, nitro, azide, phosphonyl, phosphinyl, oxo, imine, oxime, hydrazone, carbonyl, thiocarbonyl, a urea group, a thiourea group, O-carbamyl, N-carbamyl, O-thiocarbamyl, N-thiocarbamyl, S-thiocarbamyl, C-amido, N-amido, C-carboxy, O-carboxy, sulfonamido, guanyl, guanidinyl, hydrazine, hydrazide, thiohydrazide, and amino.

Herein, the term "alkynyl" describes an unsaturated aliphatic hydrocarbon comprise at least one carbon-carbon triple bond, including straight chain and branched chain groups. Preferably, the alkynyl group has 2 to 20 carbon atoms. More preferably, the alkynyl is a medium size alkynyl having 2 to 10 carbon atoms. Most preferably, unless otherwise indicated, the alkynyl is a lower alkynyl having 2 to 4 carbon atoms. The alkynyl group may be substituted or non-substituted. Substituted alkynyl may have one or more substituents, whereby each substituent group can independently be, for example, cycloalkyl, alkenyl, aryl, heteroaryl, heteroalicyclic, halo, hydroxy, alkoxy, aryloxy, thiohydroxy, thioalkoxy, thioaryloxy, sulfinyl, sulfonyl, sulfonate, sulfate, cyano, nitro, azide, phosphonyl, phosphinyl, oxo, imine, oxime, hydrazone, carbonyl, thiocarbonyl, a urea group, a thiourea group, O-carbamyl, N-carbamyl, O-thiocarbamyl, N-thiocarbamyl, S-thiocarbamyl, C-amido, N-amido, C-carboxy, O-carboxy, sulfonamido, guanyl, guanidinyl, hydrazine, hydrazide, thiohydrazide, and amino.

A "cycloalkyl" group refers to a saturated on unsaturated all-carbon monocyclic or fused ring (i.e., rings which share an adjacent pair of carbon atoms) group wherein one of more of the rings does not have a completely conjugated pi-electron system. Examples, without limitation, of cycloalkyl groups are cyclopropane, cyclobutane, cyclopentane, cyclopentene, cyclohexane, cyclohexadiene, cycloheptane, cycloheptatriene, and adamantane. A cycloalkyl group may be substituted or non-substituted. When substituted, the substituent group can be, for example, alkyl, alkenyl, alkynyl, cycloalkyl, aryl, heteroaryl, heteroalicyclic, halo, hydroxy, alkoxy, aryloxy, thiohydroxy, thioalkoxy, thioaryloxy, sulfinyl, sulfonyl, sulfonate, sulfate, cyano, nitro, azide, phosphonyl, phosphinyl, oxo, imine, oxime, hydrazone, carbonyl, thiocarbonyl, a urea group, a thiourea group, O-carbamyl, N-carbamyl, O-thiocarbamyl, N-thiocarbamyl, S-thiocarbamyl, C-amido, N-amido, C-carboxy, O-carboxy, sulfonamido, guanyl, guanidinyl, hydrazine, hydrazide, thiohydrazide, and amino, as these terms are defined herein. When a cycloalkyl group is unsaturated, it may comprise at least one carbon-carbon double bond and/or at least one carbon-carbon triple bond.

An "aryl" group refers to an all-carbon monocyclic or fused-ring polycyclic (i.e., rings which share adjacent pairs of carbon atoms) having a completely conjugated pi-electron system. Examples, without limitation, of aryl groups are phenyl, naphthalenyl and anthracenyl. The aryl group may be substituted or non-substituted. When substituted, the substituent group can be, for example, alkyl, alkenyl, alkynyl, cycloalkyl, aryl, heteroaryl, heteroalicyclic, halo, hydroxy, alkoxy, aryloxy, thiohydroxy, thioalkoxy, thioaryloxy, sulfinyl, sulfonyl, sulfonate, sulfate, cyano, nitro, azide, phosphonyl, phosphinyl, oxo, imine, oxime, hydrazone, carbonyl, thiocarbonyl, a urea group, a thiourea group, O-carbamyl, N-carbamyl, O-thiocarbamyl, N-thiocarbamyl, S-thiocarbamyl, C-amido, N-amido, C-carboxy, O-carboxy, sulfonamido, guanyl, guanidinyl, hydrazine, hydrazide, thiohydrazide, and amino, as these terms are defined herein.

A "heteroaryl" group refers to a monocyclic or fused ring (i.e., rings which share an adjacent pair of atoms) having in the ring(s) one or more atoms, such as, for example, nitrogen, oxygen and sulfur and, in addition, having a completely conjugated pi-electron system. Examples, without limitation, of heteroaryl groups include pyrrole, furan, thiophene, imidazole, oxazole, thiazole, pyrazole, pyridine, pyrimidine, quinoline, isoquinoline and purine. The heteroaryl group may be substituted or non-substituted. When substituted, the substituent group can be, for example, alkyl, alkenyl, alkynyl, cycloalkyl, aryl, heteroaryl, heteroalicyclic, halo, hydroxy, alkoxy, aryloxy, thiohydroxy, thioalkoxy, thioaryloxy, sulfinyl, sulfonyl, sulfonate, sulfate, cyano, nitro, azide, phosphonyl, phosphinyl, oxo, imine, oxime, hydrazone, carbonyl, thiocarbonyl, a urea group, a thiourea group, O-carbamyl, N-carbamyl, O-thiocarbamyl, N-thiocarbamyl, S-thiocarbamyl, C-amido, N-amido, C-carboxy, O-carboxy, sulfonamido, guanyl, guanidinyl, hydrazine, hydrazide, thiohydrazide, and amino, as these terms are defined herein.

A "heteroalicyclic" group refers to a monocyclic or fused ring group having in the ring(s) one or more atoms such as nitrogen, oxygen and sulfur. The rings may also have one or more double bonds. However, the rings do not have a completely conjugated pi-electron system. The heteroalicyclic may be substituted or non-substituted. When substituted, the substituted group can be, for example, alkyl, alkenyl, alkynyl, cycloalkyl, aryl, heteroaryl, heteroalicyclic, halo, hydroxy, alkoxy, aryloxy, thiohydroxy, thioalkoxy, thioaryloxy, sulfinyl, sulfonyl, sulfonate, sulfate, cyano, nitro, azide, phosphonyl, phosphinyl, oxo, imine, oxime, hydrazone, carbonyl, thiocarbonyl, a urea group, a thiourea group, O-carbamyl, N-carbamyl, O-thiocarbamyl, N-thiocarbamyl, S-thiocarbamyl, C-amido, N-amido, C-carboxy, O-carboxy, sulfonamido, guanyl, guanidinyl, hydrazine, hydrazide, thiohydrazide, and amino, as these terms are defined herein. Representative examples are piperidine, piperazine, tetrahydrofuran, tetrahydropyran, morpholine and the like.

An "azide" group refers to a -N=N⁺=N⁻ group.

An "alkoxy" group refers to any of an -O-alkyl, -O-alkenyl, -O-alkynyl, -O-cycloalkyl, and -O-heteroalicyclic group, as defined herein.

An "aryloxy" group refers to both an -O-aryl and an -O-heteroaryl group, as defined herein.

A "hydroxy" group refers to a -OH group.

A "thiohydroxy" or "thiol" group refers to a -SH group.

A "thioalkoxy" group refers to any of an -S-alkyl, -S-alkenyl, -S-alkynyl, -S-cycloalkyl, and -S-heteroalicyclic group, as defined herein.

A "thioaryloxy" group refers to both an -S-aryl and an -S-heteroaryl group, as defined herein.

A "carbonyl" or "acyl" group refers to a -C(=O)-R' group, where R' is defined as hereinabove.

A "thiocarbonyl" group refers to a -C(=S)-R' group, where R' is as defined herein.

A "C-carboxy" group refers to a -C(=O)-O-R' group, where R' is as defined herein.

An "O-carboxy" group refers to an R'C(=O)-O- group, where R' is as defined herein.

A "carboxylic acid" group refers to a -C(=O)OH group.

An "oxo" group refers to a =O group.

An "imine" group refers to a =N-R' group, where R' is as defined herein.

An "oxime" group refers to a =N-OH group.

A "hydrazone" group refers to a =N-NR'R" group, where each of R' and R" is as defined herein.

A "halo" group refers to fluorine, chlorine, bromine or iodine.

A "sulfinyl" group refers to an -S(=O)-R' group, where R' is as defined herein.

A "sulfonyl" group refers to an -S(=O)₂-R' group, where R' is as defined herein.

A "sulfonate" group refers to an -S(=O)₂-O-R' group, where R' is as defined herein.

A "sulfate" group refers to an -O-S(=O)₂-O-R' group, where R' is as defined as herein.

A "sulfonamide" or "sulfonamido" group encompasses both S-sulfonamido and N-sulfonamido groups, as defined herein.

An "S-sulfonamido" group refers to a -S(=O)₂-NR'R" group, with each of R' and R" as defined herein.

An "N-sulfonamido" group refers to an R'S(=O)₂-NR"- group, where each of R' and R" is as defined herein.

An "O-carbamyl" group refers to an -OC(=O)-NR'R'' group, where each of R' and R" is as defined herein.

An "N-carbamyl" group refers to an R'OC(=O)-NR''- group, where each of R' and R" is as defined herein.

An "O-thiocarbamyl" group refers to an -OC(=S)-NR'R'' group, where each of R' and R" is as defined herein.

An "N-thiocarbamyl" group refers to an R'OC(=S)NR" - group, where each of R' and R" is as defined herein.

An "S-thiocarbamyl" group refers to an -SC(=O)-NR'R'' group, where each of R' and R" is as defined herein.

An "amide" or "amido" group encompasses C-amido and N-amido groups, as defined herein.

A "C-amido" group refers to a -C(=O)-NR'R'' group, where each of R' and R" is as defined herein.

An "N-amido" group refers to an R'C(=O)-NR''- group, where each of R' and R" is as defined herein.

A "urea group" refers to an -N(R')-C(=O)-NR"R‴ group, where each of R', R" and R" is as defined herein.

A "thiourea group" refers to a -N(R')-C(=S)-NR"R‴ group, where each of R', R" and R" is as defined herein.

A "nitro" group refers to an -NO₂ group.

A "cyano" group refers to a -C≡N group.

The term "phosphonyl" or "phosphonate" describes a -P(=O)(OR')(OR'') group, with R' and R" as defined hereinabove.

The term "phosphate" describes an -O-P(=O)(OR')(OR") group, with each of R' and R" as defined hereinabove.

The term "phosphinyl" describes a -PR'R" group, with each of R' and R" as defined hereinabove.

The term "hydrazine" describes a -NR'-NR"R‴ group, with R', R", and R‴ as defined herein.

As used herein, the term "hydrazide" describes a -C(=O)-NR'-NR"R‴ group, where R', R" and R'" are as defined herein.

As used herein, the term "thiohydrazide" describes a -C(=S)-NR' -NR"R‴ group, where R', R" and R'" are as defined herein.

A "guanidinyl" group refers to an -RaNC(=NRd)-NRbRc group, where each of Ra, Rb, Rc and Rd can be as defined herein for R' and R''.

A "guanyl" or "guanine" group refers to an RaRbNC(=NRd)- group, where Ra, Rb and Rd are as defined herein.

For any of the embodiments described herein, each of the compounds described herein (including therapeutically active agents, absorption enhancers and polymers) may be in a form of a salt, for example, a pharmaceutically acceptable salt, and/or in a form of a prodrug.

As used herein, the phrase "pharmaceutically acceptable salt" refers to a charged species of the parent compound and its counter-ion, which is typically used to modify the solubility characteristics of the parent compound and/or to reduce any significant irritation to an organism by the parent compound, while not abrogating the biological activity and properties of the administered compound. A pharmaceutically acceptable salt of a compound as described herein can alternatively be formed during the synthesis of the compound, e.g., in the course of isolating the compound from a reaction mixture or re-crystallizing the compound.

In the context of some of the present embodiments, a pharmaceutically acceptable salt of the compounds described herein may optionally be an acid addition salt and/or a base addition salt.

An acid addition salt comprises at least one basic (e.g., amine and/or guanidinyl) group of the compound which is in a positively charged form (e.g., wherein the basic group is protonated), in combination with at least one counter-ion, derived from the selected acid, that forms a pharmaceutically acceptable salt. The acid addition salts of the compounds described herein may therefore be complexes formed between one or more basic groups of the compound and one or more equivalents of an acid.

A base addition salt comprises at least one acidic (e.g., carboxylic acid) group of the compound which is in a negatively charged form (e.g., wherein the acidic group is deprotonated), in combination with at least one counter-ion, derived from the selected base, that forms a pharmaceutically acceptable salt. The base addition salts of the compounds described herein may therefore be complexes formed between one or more acidic groups of the compound and one or more equivalents of a base.

Depending on the stoichiometric proportions between the charged group(s) in the compound and the counter-ion in the salt, the acid additions salts and/or base addition salts can be either mono-addition salts or poly-addition salts.

The phrase "mono-addition salt", as used herein, refers to a salt in which the stoichiometric ratio between the counter-ion and charged form of the compound is 1:1, such that the addition salt includes one molar equivalent of the counter-ion per one molar equivalent of the compound.

The phrase "poly-addition salt", as used herein, refers to a salt in which the stoichiometric ratio between the counter-ion and the charged form of the compound is greater than 1:1 and is, for example, 2:1, 3:1, 4:1 and so on, such that the addition salt includes two or more molar equivalents of the counter-ion per one molar equivalent of the compound.

An example, without limitation, of a pharmaceutically acceptable salt would be an ammonium cation or guanidinium cation and an acid addition salt thereof, and/or a carboxylate anion and a base addition salt thereof.

The base addition salts may include a cation counter-ion such as sodium, potassium, ammonium, calcium, magnesium and the like, that forms a pharmaceutically acceptable salt.

The acid addition salts may include a variety of organic and inorganic acids, such as, but not limited to, hydrochloric acid which affords a hydrochloric acid addition salt, hydrobromic acid which affords a hydrobromic acid addition salt, acetic acid which affords an acetic acid addition salt, ascorbic acid which affords an ascorbic acid addition salt, benzenesulfonic acid which affords a besylate addition salt, camphorsulfonic acid which affords a camphorsulfonic acid addition salt, citric acid which affords a citric acid addition salt, maleic acid which affords a maleic acid addition salt, malic acid which affords a malic acid addition salt, methanesulfonic acid which affords a methanesulfonic acid (mesylate) addition salt, naphthalenesulfonic acid which affords a naphthalenesulfonic acid addition salt, oxalic acid which affords an oxalic acid addition salt, phosphoric acid which affords a phosphoric acid addition salt, toluenesulfonic acid which affords a p-toluenesulfonic acid addition salt, succinic acid which affords a succinic acid addition salt, sulfuric acid which affords a sulfuric acid addition salt, tartaric acid which affords a tartaric acid addition salt and trifluoroacetic acid which affords a trifluoroacetic acid addition salt. Each of these acid addition salts can be either a mono-addition salt or a poly-addition salt, as these terms are defined herein.

As used herein, the term "prodrug" refers to a compound which is converted in the body to an active compound (e.g., the compound of the formula described hereinabove). A prodrug is typically designed to facilitate administration, e.g., by enhancing absorption. A prodrug may comprise, for example, the active compound modified with ester groups, for example, wherein any one or more of the hydroxyl groups of a compound is modified by an acyl group, optionally (C₁₋₄)-acyl (e.g., acetyl) group to form an ester group, and/or any one or more of the carboxylic acid groups of the compound is modified by an alkoxy or aryloxy group, optionally (C₁₋₄)-alkoxy (e.g., methyl, ethyl) group to form an ester group.

Further, each of the compounds described herein (including therapeutically active agents, absorption enhancers and polymers), including the salts thereof, can be in a form of a solvate or a hydrate thereof.

The term "solvate" refers to a complex of variable stoichiometry (e.g., di-, tri-, tetra-, penta-, hexa-, and so on), which is formed by a solute (the heterocyclic compounds described herein) and a solvent, whereby the solvent does not interfere with the biological activity of the solute.

The term "hydrate" refers to a solvate, as defined hereinabove, where the solvent is water.

The compounds described herein can be used as polymorphs and the present embodiments further encompass any isomorph of the compounds and any combination thereof.

The compounds and structures described herein encompass any stereoisomer, including enantiomers and diastereomers, of the compounds described herein, unless a particular stereoisomer is specifically indicated.

As used herein, the term "enantiomer" refers to a stereoisomer of a compound that is superposable with respect to its counterpart only by a complete inversion/reflection (mirror image) of each other. Enantiomers are said to have "handedness" since they refer to each other like the right and left hand. Enantiomers have identical chemical and physical properties except when present in an environment which by itself has handedness, such as all living systems. In the context of the present embodiments, a compound may exhibit one or more chiral centers, each of which exhibiting an (R) or an (S) configuration and any combination, and compounds according to some embodiments of the present invention, can have any their chiral centers exhibit an (R) or an (S) configuration.

The term "diastereomers", as used herein, refers to stereoisomers that are not enantiomers to one another. Diastereomerism occurs when two or more stereoisomers of a compound have different configurations at one or more, but not all of the equivalent (related) stereocenters and are not mirror images of each other. When two diastereomers differ from each other at only one stereocenter they are epimers. Each stereo-center (chiral center) gives rise to two different configurations and thus to two different stereoisomers. In the context of the present invention, embodiments of the present invention encompass compounds with multiple chiral centers that occur in any combination of stereo-configuration, namely any diastereomer.

As used herein the term "about" refers to ± 10 %.

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention in a non-limiting fashion.

### MATERIALS AND METHODS

### Materials:

Croscarmellose sodium (Parteck^{®} CCS; crosslinked; CAS No. 74811-65-7) was obtained from Merck.

Sodium alginate (CAS No. 9005-38-3), average MW 300-350 kDa, viscosity at room temperature 350-500 mPa (non-crosslinked), was obtained from Carl Roth.
Sodium carboxymethylcellulose (CMC), CAS No. 9004-32-4 (non-crosslinked), low viscosity (43 mPa at room temperature) was obtained from Merck or Ashland.

Sodium starch glycolate Type A (Primojel^{®}; cross-linked; CAS No. 9063-38-1), average MW 2,000 kDa, viscosity at room temperature lower than 200 mPa, was obtained from DFE Pharma.

### pH determination using pH meter:

Test substances were dissolved in various volumes of 0.1 M HCl solution (pH 1.2) at concentrations of 1, 2, 4, 10, 20, 50, 100 and 150 mg/ml. The solution pH was determined using an MP-103 pH-meter (MRC, Israel) equipped with ELC-10-00 electrode (MRC, Israel) or with a thin electrode HI1083 (HANNA instruments Inc.), shortly after dissolution and up to 26 hours later in order to ensure that the pH remained stable. The highest pH value obtained during the experimental time period was recorded in order to compare the acid-neutralizing capacity of each substance.

Other pH measurements were performed by adding a suitable colorimetric pH indicator solution to the tested medium.

### EXAMPLE 1

### Effect of basic polymers on pH in simulated gastric fluid

The effects of basic polymers on gastric fluid and on the behavior of SNAC in gastric fluid was investigated using HCl solutions as simulated gastric fluid.

SNAC and the basic polymers sodium alginate, sodium carboxymethylcellulose in non-crosslinked (Na-CMC) and crosslinked (croscarmellose sodium, CCS) forms, and sodium starch glycolate (SSG) were dissolved in various volumes of 0.1 M HCl solution (pH 1.2) at concentrations of 1, 2, 4, 10, 20, 50, 100 and 150 mg/ml, and the solution pH was determined using an MP-103 pH-meter (MRC, Israel) equipped with ELC-10-00 electrode (MRC, Israel) , as described in the Materials and Methods section hereinabove.

As shown in FIG. 1, upon addition to a HCl solution (pH 1.2), SNAC dissolved rapidly and elevated the pH in a concentration-dependent manner, wherein the pH 5 minutes after dissolution was about 1.5 upon addition of 10 mg/ml SNAC, 6.5 upon addition of 20 mg/ml SNAC, and about 7.5 upon addition of 100 mg/ml SNAC.

These results indicate that a substantial proportion of SNAC is converted under gastric conditions from a salt to the corresponding carboxylic acid (NAC), which is substantially less water soluble than the salt and which is not an effective permeation enhancer.

The effect of pH on peptide proteolysis by pepsin was determined by incubating 13.5 µg/ml human parathyroid hormone (1-34) (hPTH(1-34)) with 150 µg/ml pepsin, at various pH values, and determining the percentage of hPTH(1-34) which remained. The amount of hPTH(1-34) remaining upon addition to 150 µg/ml pepsin at 37 °C was determined in media with different pH values, followed by immediate (10 second) vortex and centrifugation for 3 minutes at 4 °C, 4000 RCF. The following media were used: simulated gastric fluid (pH 2), prepared from 0.01 M HCl and NaCl; phthalate buffer (pH 4 or 5); and phosphate buffer (pH 6 or 7).

As shown in FIG. 2, proteolysis of hPTH(1-34) was inhibited when the pH increased to about pH 6 or higher.

Taken together, the above results indicate that SNAC can inhibit peptide degradation by pepsin under gastric conditions by raising the pH, at the cost of loss of at least a portion of the active salt form of the absorption enhancer.

In order to obtain a beneficial rise in pH with a reduced loss of the active form of the absorption enhancer, the effect of various concentrations of different polymers containing carboxylate groups (sodium alginate, sodium starch glycolate, sodium carboxymethylcellulose, and croscarmellose sodium) on pH upon addition to a 0.1 M HCl solution (pH 1.2) was also evaluated. FIG. 3A presents pH values determined using an MP-103 pH-meter (MRC, Israel) equipped with ELC-10-00 electrode (MRC, Israel)and FIG. 3B presents pH values determined using an MP-103 pH-meter (MRC, Israel) equipped with a thin electrode HI1083 (HANNA instruments Inc.).

As shown in FIGs. 3A and 3B, each of the tested polymers exhibited a concentration-dependent increase in pH, with pH values of about 4-4.5 being obtained at the higher concentrations tested.

The sodium alginate, Na-CMC, CCS and sodium starch glycolate tested powders form gels (of various viscosities) upon addition to aqueous media, and the dispersion of the polymers in such gels may be inhomogeneous. In order to assess whether the acid-neutralizing capacity of the polymer is determined by the concentration of the polymer in gel, preliminary studies were performed with polymers in a powder form inserted to the acidic medium without mixing. pH values were determined using a suitable pH indicator solution.

The polymers in powder form produced local acid-neutralizing effects: sodium starch glycolate dissolved quickly with strong acid-neutralization, croscarmellose sodium dissolved quickly with weaker acid-neutralization, sodium carboxymethylcellulose did not dissolve well but exhibited some acid-neutralization, and sodium alginate exhibited little acid-neutralization (data not shown).

These results indicate that of the tested polymers, sodium starch glycolate (and to some extent, croscarmellose sodium) has a particularly strong local acid-neutralizing effect in powder form, rendering it particularly suitable for non-enterically coated formulations with an absorption enhancer such as SNAC.

Without being bound by any particular theory, it is believed that the limited effect of sodium carboxymethylcellulose and sodium alginate is associated with slow penetration of the water into the polymeric matrix, whereas the particularly effective acid neutralization of sodium starch glycolate is at least partially associated with its rapid water absorption (swelling).

As sodium starch glycolate and croscarmellose sodium exhibited the strongest local acid-neutralizing capacities, additional experiments were performed with these polymers.

To assess the maximal acid-neutralizing capacity of the polymers, experiments were repeated with each polymer compressed to a form of a tablet.

In preliminary studies, flat round 100 mg tablets were formed from sodium starch glycolate or croscarmellose sodium using a 6 mm punch and 2 ton compression force, and the tablets were placed into 40 ml HCl solutions (static dissolution) at various pH levels - 1.2, 1.5 or 2.0. A temperature of 37 °C was maintained, using a water bath. The resulting pH values were determined using a suitable pH indicator solution.

Although sodium starch glycolate and croscarmellose sodium tablets exhibited different swelling patterns, they exhibited comparable neutralization strength in HCl solutions (data not shown). Sodium starch glycolate tablets resulted in stronger acid neutralization than did croscarmellose sodium tablets for each tested initial pH (wherein the efficacy of local acid neutralization was greater when the initial pH was higher).

These results indicate that although sodium starch glycolate and croscarmellose sodium may have comparable efficacy in increasing the pH of gastric fluid to at least about 4, sodium starch glycolate is more effective than croscarmellose sodium at raising the pH of gastric fluid to at least about 5, indicating that sodium starch glycolate is more effective at acid neutralization than is croscarmellose sodium.

In additional studies, 100 mg sodium starch glycolate tablets were prepared with an 8 mm round punch and 1.5 ton compression force. The tablets were dissolved without shaking in weighing boats containing 50 ml 0.01 M HCl solutions (pH 2.0, 37 °C), and local pH was determined.

Determination of pH in the proximity of the tablets, by adding an indicator to the solution, indicated that the local pH was about 7.

These results indicate that sodium starch glycolate increases local pH to well above pH 5 (the pKa of SNAC), thereby facilitating dissolution of SNAC at concentrations effective for the action of SNAC and related compounds as an absorption enhancer, as well as inhibiting peptide degradation by pepsin (which is active only at low pH).

Without being bound by any particular theory, it is believed that the local nature of the observed pH increase is associated with the viscous gel which forms upon polymer dissolution (hydration and relaxation upon wetting), which limits the transport of acid from outside to the local region with higher pH. It is further believed that due to this inhomogeneity, determination of local pH presents a more relevant parameter than pH of the medium as a whole. For example, the generally smaller increases in pH of the medium as a whole (FIGs. 3A-B) may be explained as reflecting an average of the pH of the region proximal to the tablets in which acid is neutralized effectively and the pH of distal regions in which pH is less affected.

In additional studies, 100 mg tablets were formed from various polymers (e.g., sodium starch glycolate, croscarmellose sodium, sodium carboxymethylcellulose and sodium alginate) using an 8 mm round punch and 2 ton compression force. The effects of the tablets on local pH were compared upon placement of the tablets in 0.01 M HCl solution (pH 2.0, 37 °C). pH was determined by adding an indicator to the solution.

The sodium starch glycolate tablet rapidly swelled and disintegrated, and produced a strong local acid-neutralizing effect; the croscarmellose sodium tablets also rapidly disintegrated, but produced a weaker local acid-neutralizing effect; the sodium carboxymethylcellulose tablets swelled slowly and exhibited some local acid-neutralization; and the sodium alginate tablets remained intact and exhibited little local acid-neutralization (data not shown).

In addition, a control tablet prepared with hydroxypropyl methylcellulose (HPMC), a polymer with no anionic groups, was tested in a similar manner, and exhibited no observable effect on pH.

Because of the gradual hydration of the tablets, the acid-neutralizing effect of the polymers in the swollen matrix may be inhomogeneous. The pH at the different locations of the tablet matrix was therefore assessed in additional sets of experiments, as follows.

200 mg tablets were prepared from sodium alginate, Na-CMC, CCS or sodium starch glycolate (SSG) with a 10 mm round punch and 1 ton compression force. Each tablet was placed at the center of a dish containing 100 ml 0.01 M HCl solutions (pH 2.0, room temperature), and after hydration, local pH was determined at four different points of the swollen tablet matrix, as shown in FIGs. 6A (for SSG), 6B (for CCS), 6C (for Na-CMC; CMC-Na) and 6D (for sodium alginate; Alg-Na), and as follows: (i) in the HCl solution outside of the formed gel boundaries (about 2-3 cm from the swollen matrix's surface); (ii) at the surface of the swollen matrix; (iii) at an intermediate distance between the surface and the center of the swollen matrix (about 0.5-1 cm from each of the surface and the center); and (iv) at the center of the swollen matrix ). Measurement of the local pH inside the tablets was enables by using a pH meter with a thin electrode (as described under the "Materials and Methods" section).

As shown in FIGs. 6A-D, the SSG tablet rapidly swelled and disintegrated, and produced a strong local acid-neutralizing effect reaching pH 5.7 in the center of the swollen matrix (FIG. 6A). The CCS tablet also rapidly swelled, and produced a slightly weaker local acid-neutralizing effect (FIG. 6B). The CMC-Na tablet swelled slowly and exhibited strong local acid-neutralization (FIG. 6C); and the Alg-Na tablet exhibited the slowest swelling rate and local acid-neutralization similar to CCS (FIG. 6D). For all the tested polymers, the pH values measured in the center of the swollen matrix was higher than other areas, further supporting a concentration-dependent acid-neutralizing capacity of the polymers.

The results of these two sets of experiments show that for all the tested alkaline polymers, an increase in the local pH inside the matrix is achieved, up to pH of about 5-6. Such pH values are in line with the desired pH for maintaining SNAC in a dissolvable salt form, as the pKa of SNAC is about 5. This pH increase thus reduces precipitation of SNAC and consequently achieves higher concentrations of dissolved SNAC which are required for its action (and other absorption enhancers) as an absorption enhancer. The local pH increase also inhibits peptide degradation by pepsin (which is active only at low pH). These results further indicate that an alkaline (basic) polymer, by exhibiting limited diffusion in acidic solution and thus affecting pH primarily in a local manner, can result in a stronger and/or more prolonged effect on pH.

In yet another set of experiments, SSG, as an example of a fast swelling polymer that produces a high local pH, and CMC-Na, as an example of slow swelling polymer that produces a high local pH, were further tested in porcine gastric juice (withdrawn endoscopically from a sedated adult female domestic pig, *sus scrofa domesticus*). 200 mg tablets were prepared from each polymer with a 10 mm round punch and 1 ton compression force. Each tablet was placed at the center of a dish containing 100 ml of the porcine gastric juice at room temperature, and after hydration (wetting), local pH was determined at four different points of the swollen tablet matrix (i)-(iv) as explained above for the data shown in FIGs. 6A-D. The results are presented in FIG. 7A for SSG and in FIG. 7B for CMC-Na and are similar to those shown in FIGs. 6A and 6C, respectively.

Taken together, the above results indicate that alkaline group-containing polymers, especially sodium starch glycolate and croscarmellose sodium, can effectively neutralize acid within a local region (e.g., within the stomach), and thereby reduce protonation and inactivation of SNAC, as well as degradation of peptides by pepsin, within such a region. This phenomenon would be of particular importance when the polymer, SNAC and a peptide or polypeptide are co-localized (e.g., within a single dosage form), allowing prolonged SNAC-induced absorption of the peptide or the polypeptide.

### EXAMPLE 2

### Effect of sodium starch glycolate on peptide absorption in vivo rat model

The ability of sodium starch glycolate (SSG) to facilitate drug absorption was assessed in an *in vivo* rat model, using teriparatide (hPTH(1-34)) as a model peptide drug and SNAC as model absorption enhancer.

Formulations were prepared with compositions presented in Table 1. All substances were in a dry powder form and mixed geometrically using mortar and pestle. Afterwards, mini-tablets were prepared from the mixes using a 2 mm punch and die set at 0.5 ton/cm².

**Table 1: Compositions of exemplary formulations (comprising 90 µg hPTH(1-34), about 14.4 mg SNAC, and about 0.15 mg magnesium stearate) administered to rats in different test groups**

| **Formulation** | **Soybean trypsin inhibitor (mg)** | **Sodium starch glycolate (mg)** | **Total dose weight (mg)** | **No. of rats** |
|---|---|---|---|---|
| SNAC + SBTI + 20 % SSG | about 0.5 | 3.6 | 18.6 | 6 |
| SNAC + 30 % SSG | 0 | 4.37 | 18.9 | 5 |
| SNAC + SBTI | about 0.5 | 0 | 15.0 | 7 |
| SNAC | 0 | 0 | 14.6 | 8 |

Male Wistar rats (250-300 grams) were divided into groups which received different formulations (as described in Table 1); and were deprived of food the night before the experiments and during the experiment, and deprived of water an hour before the experiment and during the experiment. Administration of the mini-tablets to the rats was performed using a customized intragastric gavage. Blood was taken from the cheek at predetermined time points. Plasma was separated and hPTH(1-34) quantified by a commercially available Elisa kit for the determination of hPTH(1-34) in plasma or cell culture media (Quidel Corp. Athens, OH, USA; Cat. No. 60-3900).

As shown in FIG. 4 and in Table 2 below, inclusion of 20 % sodium starch glycolate in exemplary formulations with SBTI resulted in about a 1.5-fold greater median Cmax and AUC (area under curve), as compared to the corresponding formulation without sodium starch glycolate.

As shown in FIG. 5 and in Table 2 below, inclusion of 30 % sodium starch glycolate in exemplary formulations (without SBTI) resulted in about a 4-fold greater median Cmax and AUC (area under curve), as compared to the corresponding formulation without sodium starch glycolate.

As further shown in FIGs. 4 and 5, sodium starch glycolate increased plasma levels of hPTH(1-34) during the elimination phase, indicating prolonged absorption.

**Table 2: Pharmacokinetic parameters for hPTH(1-34) (median with range) of exemplary formulations following oral administration of formulations to male Wistar rats**

| **Formulation** | **Cmax (pg/ml)** | **AUC(0-last) (minutes*pg/ml)** | **Tmax (minutes)** | **No. of rats** |
|---|---|---|---|---|
| SNAC + SBTI + 20 % SSG | 1025 (154-2990) | 23182 (3731-62856) | 10 (5-30) | 6 |
| SNAC + 30 % SSG | 1633 (330-20652) | 38015 (7001-414106) | 20 (10-45) | 5 |
| SNAC + SBTI | 739 (92-1240) | 14151 (1949-34046) | 15 (5-15) | 7 |
| SNAC | 456 (212-1590) | 8474 (4416-25311) | 10 (10-30) | 8 |

These results support those of Example 1, and confirm the ability of sodium starch glycolate to markedly increase absorption of a polypeptide co-administered with an absorption enhancer such as SNAC, and modulate the pharmacokinetic profile, both in the presence and absence of a protease inhibitor.

### EXAMPLE 3

### Effect of sodium starch glycolate on peptide absorption in an in vivo pig model

The ability of sodium starch glycolate (SSG) to facilitate drug absorption was further assessed in an *in vivo* pig model, which allows testing of unit dosage forms with conventional dimensions for use in humans, using teriparatide (hPTH(1-34)) as a model peptide drug and SNAC as model absorption enhancer.

Formulations with or without 25 % SSG were prepared with compositions presented in Table 3. All substances were in a dry powder form and mixed geometrically using mortar and pestle. Afterwards, tablets were prepared from the mixes using a manual press.

The obtained tablets were orally administered to female Sinclair minipigs (18-20 kg) on different visits. Pigs were deprived of food the night before the experiments and during the experiment and deprived of water an hour before the experiment and during the experiment. Administration was facilitated by a customized device for oral administration accompanied by about 30 ml of water. Blood was taken from the jugular vein through an indwelling cannula at predetermined time points. Plasma was separated and hPTH(1-34) was quantified using a commercially available Elisa kit for the determination of hPTH(1-34) in plasma or cell culture media (Quidel Corp. Athens, OH, USA; Cat# 60-3900).

**Table 3: Compositions of exemplary formulations administered to pigs, comprising teriparatide (hPTH(1-34)), SNAC and magnesium stearate, with or without 25 % SSG**

| **Formulation** | **hPTH(1-34) (mg)** | **SNAC (mg)** | **Mg Stearate (mg)** | **SSG (mg)** | **Total weight (mg)** |
|---|---|---|---|---|---|
| SNAC 1 tablet (8 mm) | 1 | 166 | 0.17 | --- | 167 |
| SNAC + SSG 1 tablet (8 mm) | 1 | 166 | 0.17 | 55 | 222 |
| SNAC 22 tablets (2 mm) | 1 | 166 | 0.17 | --- | 167 |
| SNAC + SSG 22 tablets (2 mm) | 1 | 166 | 0.17 | 55 | 222 |

As shown in Table 4, formulations containing sodium starch glycolate consistently produced higher Cmax and AUC values than the corresponding formulations without sodium starch glycolate, with plasma levels remaining higher also during the elimination phase.

These results further confirm the ability of an alkaline group-containing polymer to enhance absorption of a polypeptide in the presence of an absorption enhancer.

**Table 4: Teriparatide (hPTH(1-34)) pharmacokinetic parameters (median ± SEM) of exemplary formulations following oral administration to female Sinclair minipigs (a. p<0.05 compared to the counterpart group by Wilcoxon signed rank test)**

| **Formulation** | **hPTH(1-34) (mg)** | **Cmax (pg/ml)** | **AUC(0-last) (min*pg/ml)** | **N (pigs)** |
|---|---|---|---|---|
| SNAC 1 tablet (8 mm) | 1 | about 95 | about 1480 | 6 |
| SNAC + SSG 1 tablet (8 mm) | 1 | about 160 | about 5490^{a} | 6 |
| SNAC 22 tablets (2 mm) | 1 | about 33 | about 500 | 4 |
| SNAC + SSG 22 tablets (2 mm) | 1 | about 58 | about 1300 | 4 |

## Claims

1. A pharmaceutical composition comprising, as a therapeutically active agent, a parathyroid hormone; and an absorption enhancer, and a polymer comprising a plurality of alkaline groups, wherein a concentration of said polymer in the composition is at least 10 weight percent of the total weight of the composition, said absorption enhancer being a substituted or non-substituted fatty acid or a salt thereof.

2. The composition of claim 1, wherein at least a portion of said alkaline groups are carboxylate groups.

3. The composition of claim 2, wherein at least a portion of said carboxylate groups are in a form of a pharmaceutically acceptable salt.

4. The composition of any one of claims 1 to 3, wherein said absorption enhancer comprises NAC or a salt thereof.

5. The composition of any one of claims 1 to 4, wherein a concentration of said absorption enhancer is at least 50 weight percent.

6. The composition of any one of claims 1 to 5, wherein a concentration of said polymer is at least 20 weight percent.

7. The composition of any one of claims 1 to 6, wherein a total concentration of said absorption enhancer and said polymer is at least 80 weight percent.

8. The composition of any one of claims 1 to 7, wherein a concentration of said alkaline groups in the composition is at least 0.1 millimoles per gram.

9. The composition of any one of claims 1 to 8, wherein said polymer is a crosslinked polymer.

10. The composition of any one of claims 1 to 9, wherein said polymer comprises a polysaccharide.

11. The composition of any one of claims 1 to 10, wherein said polymer is **characterized by** a pKa in a range of from 1.2 to 7.5.

12. The composition of any one of claims 1 to 11, wherein said therapeutically active agent is or comprises teriparatide or eneboparatide (AZP-3601).

13. The composition of any one of claims 1 to 12, being in a form of a unit dosage form.

14. The composition of claim 13, wherein an amount of said alkaline groups in the unit dosage form is at least 0.03 millimoles.

15. The composition of any one of claims 1 to 14, being for use in the treatment of a condition treatable by said therapeutically active agent, said treatment comprising oral administration of the composition.

## Patentansprüche

1. Pharmazeutische Zusammensetzung, die als therapeutisch wirksames Mittel ein Parathormon; und einen Absorptionsverstärker und ein Polymer umfasst, das eine Vielzahl von alkalischen Gruppen umfasst, wobei eine Konzentration des Polymers in der Zusammensetzung mindestens 10 Gewichtsprozent des Gesamtgewichts der Zusammensetzung beträgt und der Absorptionsverstärker eine substituierte oder nicht substituierte Fettsäure oder ein Salz davon ist.

2. Zusammensetzung nach Anspruch 1, wobei mindestens ein Teil der alkalischen Gruppen Carboxylatgruppen sind.

3. Zusammensetzung nach Anspruch 2, wobei mindestens ein Teil der Carboxylatgruppen in Form eines pharmazeutisch verträglichen Salzes vorliegt.

4. Zusammensetzung nach einem der Ansprüche 1 bis 3, wobei der Absorptionsverstärker NAC oder ein Salz davon umfasst.

5. Zusammensetzung nach einem der Ansprüche 1 bis 4, wobei eine Konzentration des Absorptionsverstärkers mindestens 50 Gewichtsprozent beträgt.

6. Zusammensetzung nach einem der Ansprüche 1 bis 5, wobei eine Konzentration des Polymers mindestens 20 Gewichtsprozent beträgt.

7. Zusammensetzung nach einem der Ansprüche 1 bis 6, wobei eine Gesamtkonzentration des Absorptionsverstärkers und des Polymers mindestens 80 Gewichtsprozent beträgt.

8. Zusammensetzung nach einem der Ansprüche 1 bis 7, wobei eine Konzentration der alkalischen Gruppen in der Zusammensetzung mindestens 0,1 Millimol pro Gramm beträgt.

9. Zusammensetzung nach einem der Ansprüche 1 bis 8, wobei das Polymer ein vernetztes Polymer ist.

10. Zusammensetzung nach einem der Ansprüche 1 bis 9, wobei das Polymer ein Polysaccharid umfasst.

11. Zusammensetzung nach einem der Ansprüche 1 bis 10, wobei das Polymer durch einen pKa-Wert im Bereich von 1,2 bis 7,5 gekennzeichnet ist.

12. Zusammensetzung nach einem der Ansprüche 1 bis 11, wobei das therapeutisch wirksame Mittel Teriparatid oder Eneboparatid (AZP-3601) ist oder umfasst.

13. Zusammensetzung nach einem der Ansprüche 1 bis 12, die in Form einer Einzeldosierungsform vorliegt.

14. Zusammensetzung nach Anspruch 13, wobei eine Menge der alkalischen Gruppen in der Einzeldosierungsform mindestens 0,03 Millimol beträgt.

15. Zusammensetzung nach einem der Ansprüche 1 bis 14, die zur Behandlung einer Erkrankung verwendet wird, die mit dem therapeutisch wirksamen Mittel behandelt werden kann, wobei die Behandlung die orale Verabreichung der Zusammensetzung umfasst.

## Revendications

1. Composition pharmaceutique comprenant, en tant qu'agent thérapeutiquement actif, une hormone parathyroïdienne; et un activateur d'absorption, et un polymère comprenant une pluralité de groupes alcalins, où une concentration dudit polymère dans la composition est d'au moins 10 % en poids du poids total de la composition, ledit activateur d'absorption étant un acide gras substitué ou non substitué ou un sel de celui-ci.

2. Composition selon la revendication 1, où au moins une partie desdits groupes alcalins sont des groupes carboxylates.

3. Composition selon la revendication 2, où au moins une partie desdits groupes carboxylates se présente sous la forme d'un sel pharmaceutiquement acceptable.

4. Composition selon l'une quelconque des revendications 1 à 3, où ledit activateur d'absorption comprend de la NAC ou un sel de celle-ci.

5. Composition selon l'une quelconque des revendications 1 à 4, où une concentration dudit activateur d'absorption est d'au moins 50 % en poids.

6. Composition selon l'une quelconque des revendications 1 à 5, où une concentration dudit polymère est d'au moins 20 % en poids.

7. Composition selon l'une quelconque des revendications 1 à 6, où une concentration totale dudit activateur d'absorption et dudit polymère est d'au moins 80 % en poids.

8. Composition selon l'une quelconque des revendications 1 à 7, où une concentration desdits groupes alcalins dans la composition est d'au moins 0,1 millimole par gramme.

9. Composition selon l'une quelconque des revendications 1 à 8, où ledit polymère est un polymère réticulé.

10. Composition selon l'une quelconque des revendications 1 à 9, où ledit polymère comprend un polysaccharide.

11. Composition selon l'une quelconque des revendications 1 à 10, où ledit polymère est **caractérisé par** un pKa compris entre 1,2 et 7,5.

12. Composition selon l'une quelconque des revendications 1 à 11, où ledit agent thérapeutiquement actif est ou comprend du tériparatide ou de l'énéboparatide (AZP-3601).

13. Composition selon l'une quelconque des revendications 1 à 12, se présentant sous la forme d'une dose unitaire.

14. Composition selon la revendication 13, où une quantité desdits groupes alcalins dans la forme de dose unitaire est d'au moins 0,03 millimole.

15. Composition selon l'une quelconque des revendications 1 à 14, étant destinée à être utilisée dans le traitement d'une affection pouvant être traitée par ledit agent thérapeutiquement actif, ledit traitement comprenant une administration par voie orale de la composition.
